# EUROPEAN PATENT SPECIFICATION

(11) **EP 1 951 747 B1**
(45) Date of publication and mention of the grant of the patent: **01.07.2009**
(21) Application number: 06818498.5
(22) Date of filing: 13.11.2006
(51) Int. Cl.: C07K 7/56, C07K 1/113

(54) **PROCESS AND INTERMEDIATES FOR THE SYNTHESIS OF CASPOFUNGIN**
VERFAHREN UND ZWISCHENPRODUKTE ZUR SYNTHESE DES CASPOFUNGINS
PROCEDE ET INTERMEDIAIRES POUR LA SYNTHESE DE LA CASPOFUNGINE

(30) Priority: 15.11.2005 EP 05024961
(43) Date of publication of application: 06.08.2008
(73) Proprietor: SANDOZ AG, 4056 Basel (CH)
(72) Inventor: LUDESCHER, Johannes, A-6252 Breitenbach (AT); MACHER, Ingolf, A-6300 Woergl (AT); STORM, Ole, A-6330 Kufstein (AT); BERTEL, Stephan, A-6250 Kundl (AT)
(74) Representative: Wichmann, Hendrik
(86) International application number: PCT/EP2006/010867
(87) International publication number: WO 2007/057141

(56) References cited:
- WO-A-94/09033
- WO-A-94/21677
- WO-A-96/24613
- WO-A-02/083713
- BALKOVEC J.M. ET AL TETRAHEDRON LETTERS vol. 33, no. 32, 1992, pages 4529 - 4532
- ALEXAKIS A. ET AL ORGANIC LETTERS vol. 4, no. 21, 2002, pages 3611 - 3614

## Description

### Field of the Invention

The present invention relates to novel processes for preparing certain aza cyclohexapeptide compounds, novel intermediates used in said processes and a process for preparing said intermediates.

### Background of the Invention

Aza cyclohexapeptide compounds of formula I as defined below are macrocyclic lipopeptides belonging to the echinocandin family which are useful in treating systemic fungal infections, especially those caused by *Candida, Aspergillus, Histoplasma, Coccidioides* and *Blastomyces.* They have also been found useful for the treatment and prevention of infections caused by *Pneumocystis carinii* which are often found in immunocompromised patients such as those with AIDS. Pneumocandins are a subset of echinocandins which are naturally produced by the fungus *Glarea lozoyensis.* Their isolation, structure elucidation and biological evaluation have been reported by Schmatz et al in Cutaneous Antifungal Agents, 1993, pp 375-394.
Pneumocandin B₀ is a secondary metabolite produced by the fungus *Glarea lozoyensis.* previously identified as *Zalerion arboricola,* and serves as an intermediate in the production of Caspofungin (see e.g. US patents no. 5,194,377 and 5,202,309). Pneumocandin B₀ has also been named compound 1-[4,5-dihydroxy-N²-(10,12-dimethyl-1-oxotetradecyl)ornithine]-5-(3-hydroxyglutamine)-6-[3-hydroxy proline]echinocandin B, with its preferred stereoisomer being 1-[4,5-dihydroxy-N²-(10,12-dimethyl-1-oxotetradecyl)-L-ornithine]-5-(3-hydroxy-L-glutamine)-6-[3-hydroxy-L-proline]echinocandin B as is described e.g. in US patent No. 5,202,309. Pneumocandin B₀ serves as an intermediate in the production of caspofungin as is described e.g. in European Patent EP 620232.

The compound 1-[(4R,5S)-5-[(2-Aminoethyl)amino]-N²-(10,12-dimethyl-1-oxotetradecyl)-4-hydroxy-L-ornithine]-5-[(3R)-3-hydroxy-L-ornithine]-pneumocandin B₀ and its pharmaceutical acceptable salts are known under the INN caspofungin (see Merck Index, 13th edition, monograph no. 1899). Caspofungin is known to be useful for treating fungal infections and for preventing and/or treating infections caused by *Pneumocystis carinii* particularly in immunocompromised patients such as patients suffering from AIDS.

Processes for the preparation of aza cyclohexapeptide compounds and of caspofungin are described in e.g. WO 94/21677, EP 620232. WO 96/24613, US 5,552,521, WO 97/47645, US 5,936,062,and WO 02/083713.

WO 94/21677 and EP 620232 disclose processes starting from pneumocandin B₀ involving a reaction with an alkylthiol or arylthiol, e.g. aminoethylthiol, followed by oxidation to a sulfone intermediate which may subsequently be reacted with an amine compound e.g. with a diamine compound such as ethylenediamine, in an anhydrous aprotic solvent, and the reaction product may be isolated *inter alia* by chromatographic methods.

WO 96/24613 and US 5,552,521 disclose *inter alia* a process wherein pneumocandin B₀ is reduced at its primary amide function to the corresponding amine group, followed by a reaction with thiophenol and further on with ethylenediamine to obtain the aza cyclohexapeptide compound e.g. caspofungin. The yield of the reduction step is reported to be about 47% as expressed in assay yield of Compound III of WO 96/24613 or US 5,552,521.

WO 97/47645 and US 5,936,062 disclose 2 stereoselective processes starting from pneumocandin B₀. The first process comprises reduction of the primary amide function of pneumocandin B₀ to the corresponding amine group using phenylboronates as protective groups, reacting the reduced intermediate with e.g. phenylthiol and subsequently with ethylenediamine. The second process involves reduction of the primary amide function of an intermediate having an S-aryl moiety at the 5-orn position to the corresponding amine in the presence of phenylboronate as protective group followed by reaction with e.g. ethylenediamine. The reduction of the amide to the amine group is reported to have reaction yields of about 61% (HPLC assay) in both process variants.

WO 02/083713 discloses processes for preparing certain -sulfide-substituted echinocandins and/or nitrile compounds being useful as intermediates in the preparation of caspofungin. The preparation of these intermediates involves the use of boronic acid or borate for providing protective groups.

WO 94/09033 discloses a process for the synthesis of echinocandin analogues having a 5-ether functionality on the side chain of the N-terminal Orn residue.

Known processes, however, are not optimal for industrial production in terms of yield, purity, stability and amount of side-products. Some processes raise the necessity to operate under strictly anhydrous conditions, e.g. by using molecular sieves. In addition the use of protecting groups is required in some processes to achieve the desired purity. Several chromatographic steps are necessary to purify intermediates as well as the final product. Therefore, there is a need for an improved process to prepare aza cyclohexapeptide compounds in an economic way applicable in industrial scale. Additionally, it would be desirable to improve reaction yields when reducing the amide to the amine group in aza cyclohexapeptide compounds. Furthermore, there is a need for intermediates which may be isolated in a pure form, i.e. being substantially free of impurities.

### Summary of the Invention

The present invention therefore provides a process for preparing aza cyclohexapeptide compounds or pharmaceutically acceptable salts thereof in high yield and high purity. The process of the present invention is easily applicable and is able to be scaled up easily, e.g. to an industrial scale. The present invention further provides novel and highly purified intermediates to be used in said process, e.g. for the preparation of caspofungin.

Accordingly, in one aspect the present invention relates to a process for the production of aza cyclohexapeptide compounds of formula I 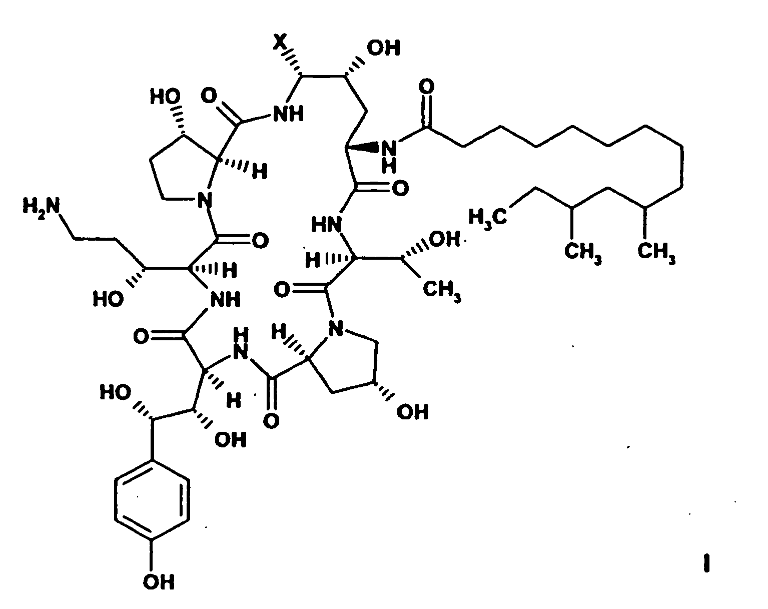 wherein X is NR₁R₂ and wherein
R₁ is H, C₁-C₈ alkyl, C₃-C₄ alkenyl, (CH₂)₂₋₄OH or (CH₂)₂₋₄NR₃R₄;
R₂ is H, C₁-C₈ alkyl, C₃-C₄alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅. (CH₂)₂O(CH₂)₂ or (CH₂)₂NH(CH₂)₂;
R₃ is H or C₁₋C₈ alkyl;
R₄ is H or C₁-C₈ alkyl;
or a pharmaceutically acceptable salt thereof comprising the steps of
a) reducing a compound of formula II or an acid addition salt thereof 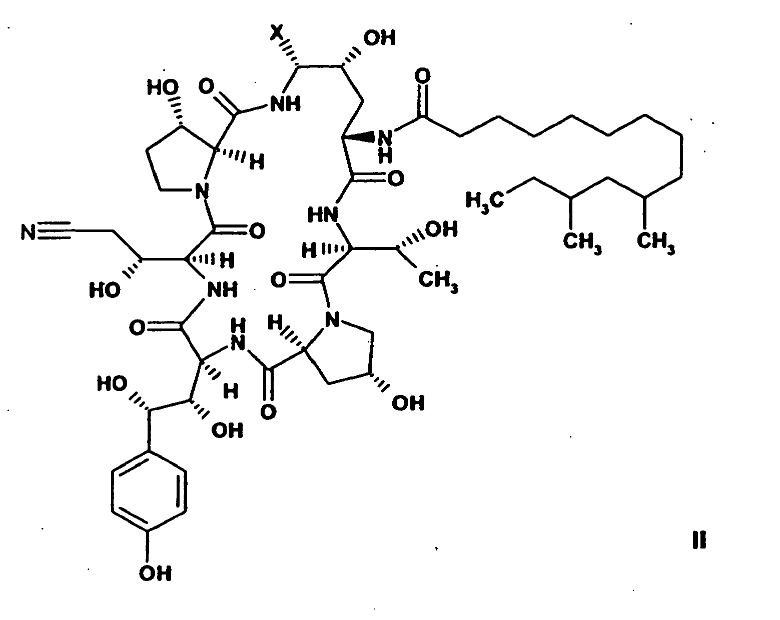 wherein X is defined as above,
   to obtain a compound of formula I or a pharmaceutically acceptable salt thereof, and
b) optionally isolating the compound of formula I or a pharmaceutically acceptable salt thereof as obtained in step a).

In another aspect the present invention provides a process for the production of a compound of formula I or a pharmaceutically acceptable salt thereof additionally comprising the steps of
a) reacting a compound of formula III 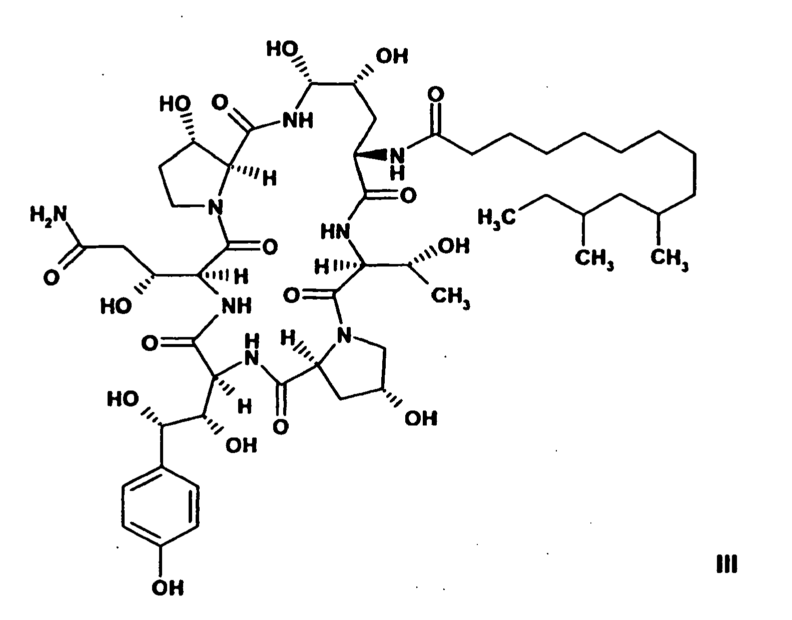 with a dehydrating agent to obtain a compound of formula IV 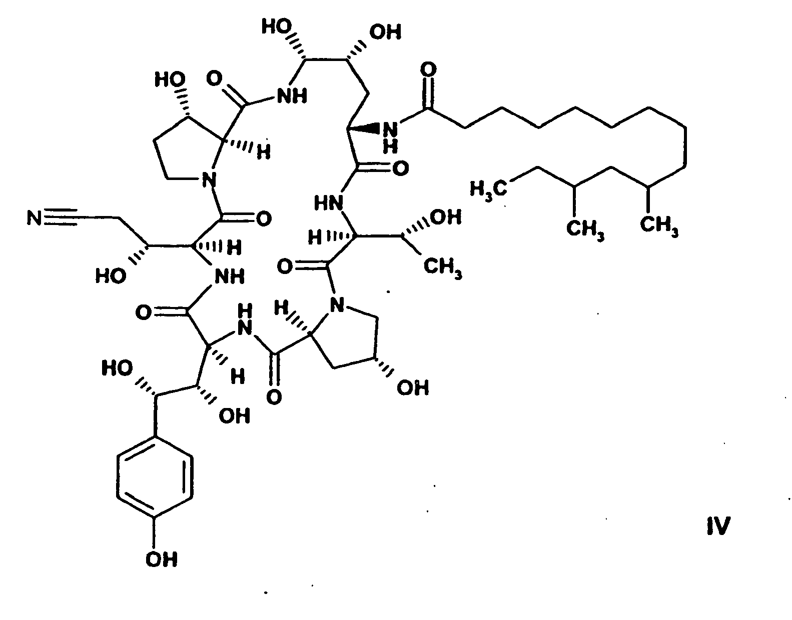
b) reacting the compound of formula IV as obtained in step a) with a thiophenol to obtain a compound of formula V 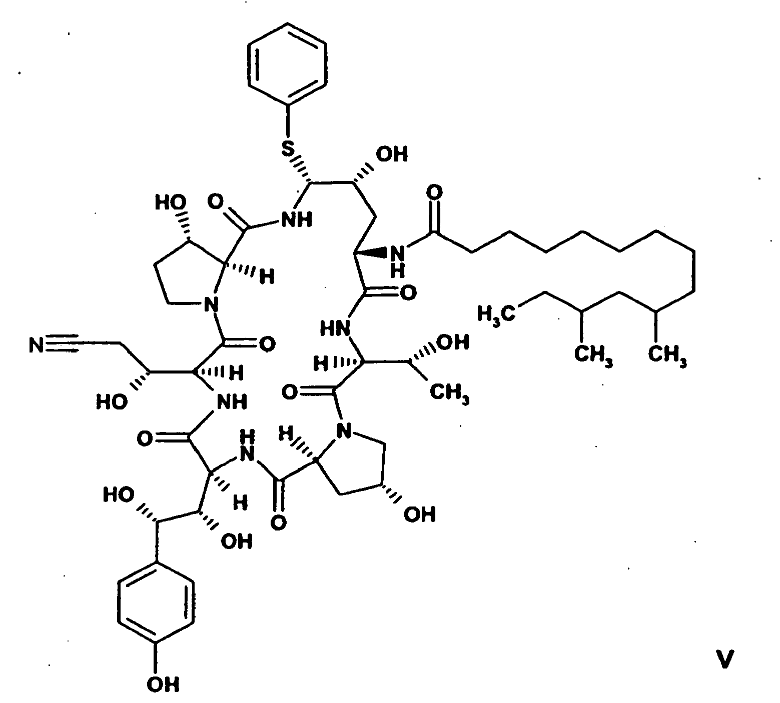
c) reacting the compound of formula V as obtained in step b) with a compound of formula HX, wherein X is as defined above, to obtain a compound of formula II or an acid addition salt thereof,
d) reducing a compound of formula II or an acid addition salt thereof as obtained in step c) to obtain a compound of formula I or a pharmaceutically acceptable salt thereof, and
e) optionally isolating the compound of formula I or a pharmaceutically acceptable salt thereof as obtained in step d).

Preferably, X is HN-CH₂-CH₂-NH₂ in the above mentioned processes.

In another aspect, the present invention provides a compound of formula II 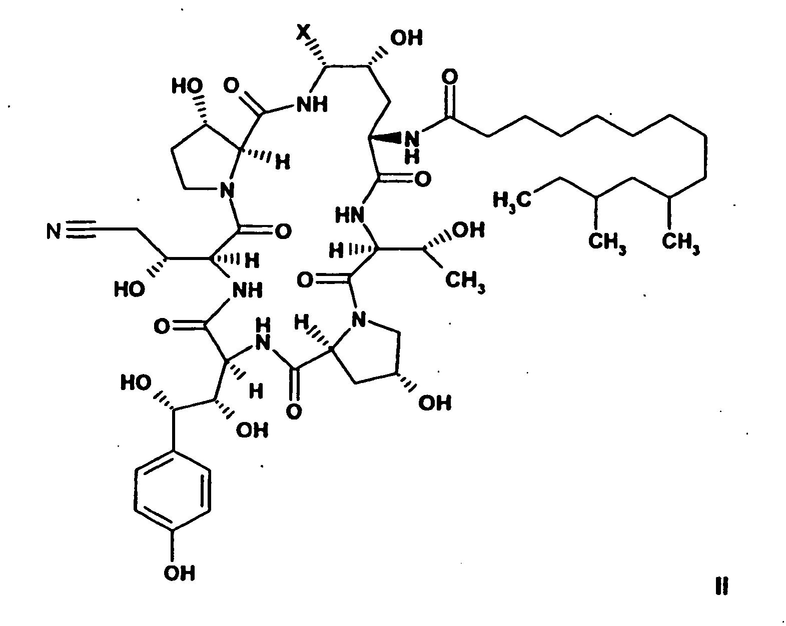 or an acid addition salt or a solvate thereof, wherein X is NR₁R₂, and wherein
R₁, is H, C₁-C₆ alkyl, C₃-C₄ alkenyl, (CH₂)₂₋₄OH or (CH₂)₂₋₄NR₃R₄;
R₂ is H, C₁-C₈ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₈ alkyl;
R₄ is H or C₁-C₈ alkyl.

Furthermore, the invention provides a compound of formula II wherein X is HN-CH₂-CH₂-NH₂ which is a compound of formula VI or an acid addition salt or a solvate thereof, preferably the monoacetate salt thereof which is compound of formula VIa.

In a further aspect, the present invention relates to a process for the production of a compound of formula II or an acid addition salt or a solvate thereof comprising the steps of
a) reacting a compound of formula III 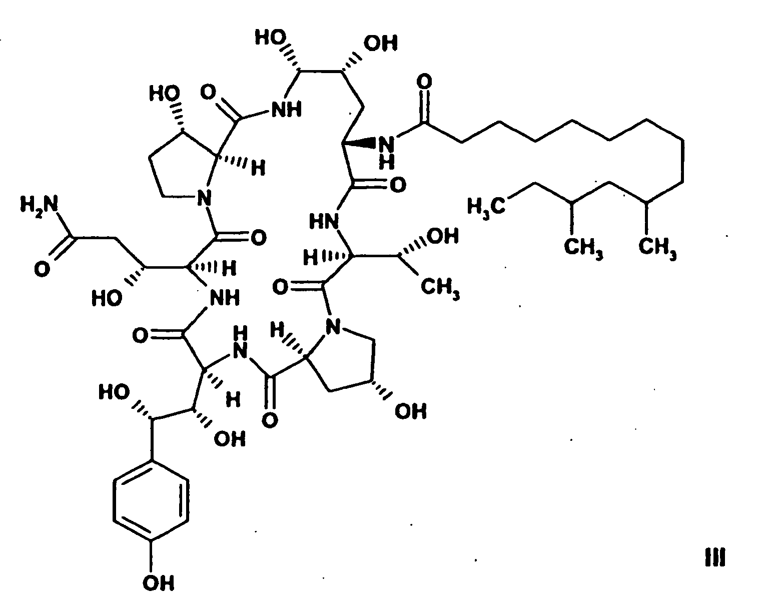 with a dehydrating agent to obtain a compound of formula IV 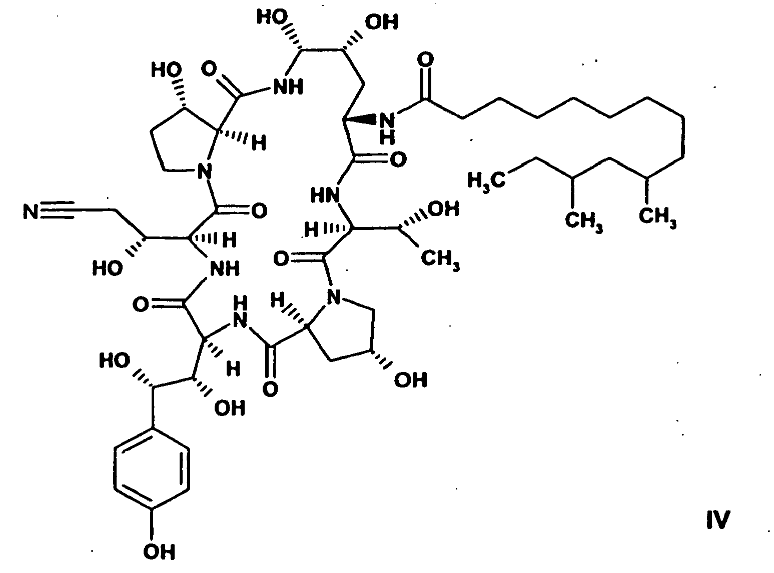
b) reacting the compound of formula IV as obtained in step a) with a thiophenol to obtain a compound of formula V 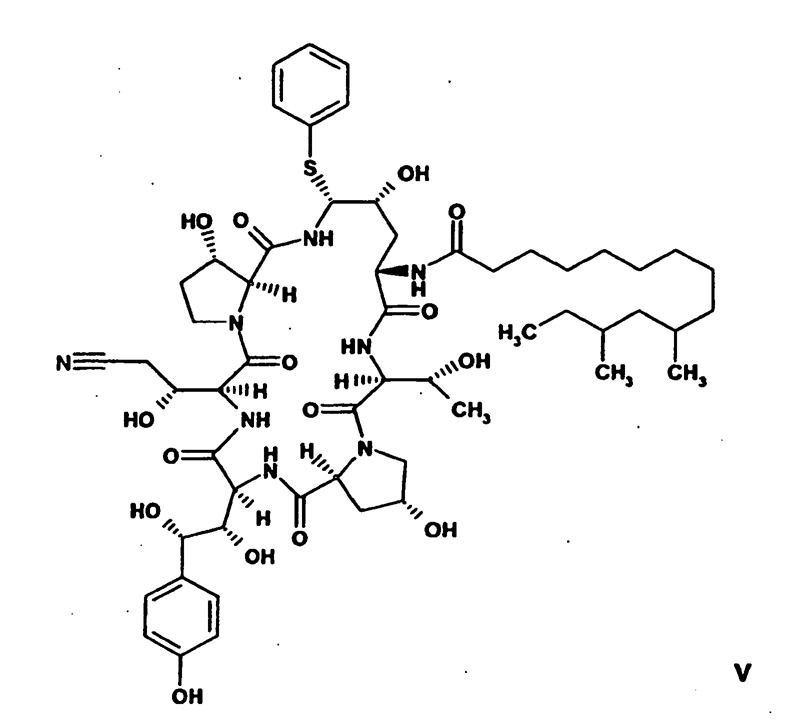
c) reacting the compound of formula V as obtained in step b) with a compound of formula HX, wherein X is NR₁R₂, and wherein
   R₁ is H, C₁-C₈ alkyl, C₃-C₄ alkenyl, (CH₂)₂₋₄OH or (CH₂)₂₋₄NR₃R₄;
   R₂ is H, C₁-C₈ alkyl, C₃-C₄ alkenyl, (CH₂)₂₋₄OH, (CH₂)₂₋₄NR₃R₄; or
   wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
   R₃ is H or C₁-C₈ alkyl;
   R₄ is H or C₁-C₈ alkyl;
   to obtain a compound of formula II or an acid addition salt or a solvate thereof, and
d) optionally isolating the compound of formula II or an acid addition salt or a solvate thereof as obtained in step c).

In an additional aspect, the present invention provides a process for the production of a compound of formula VI or an acid addition salt or a solvate thereof comprising the steps of
a) reacting a compound of formula III 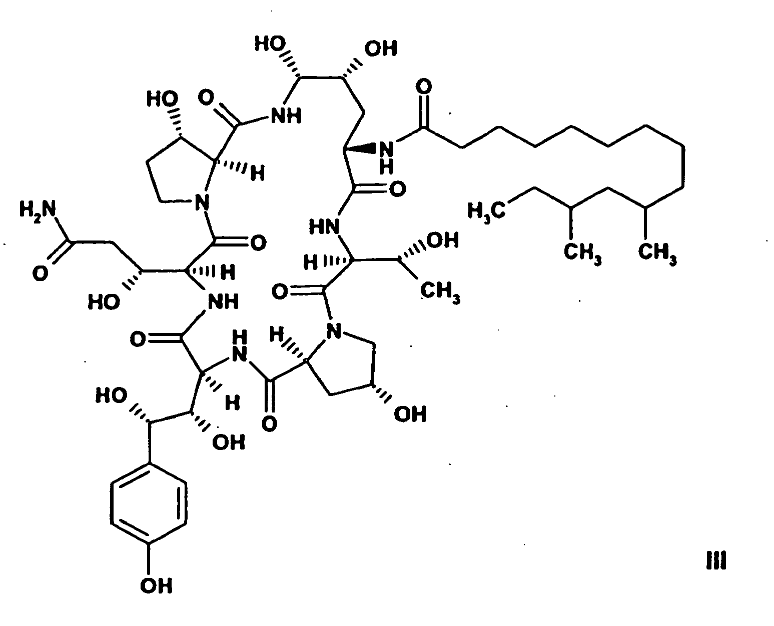 with a dehydrating agent to obtain a compound of formula IV 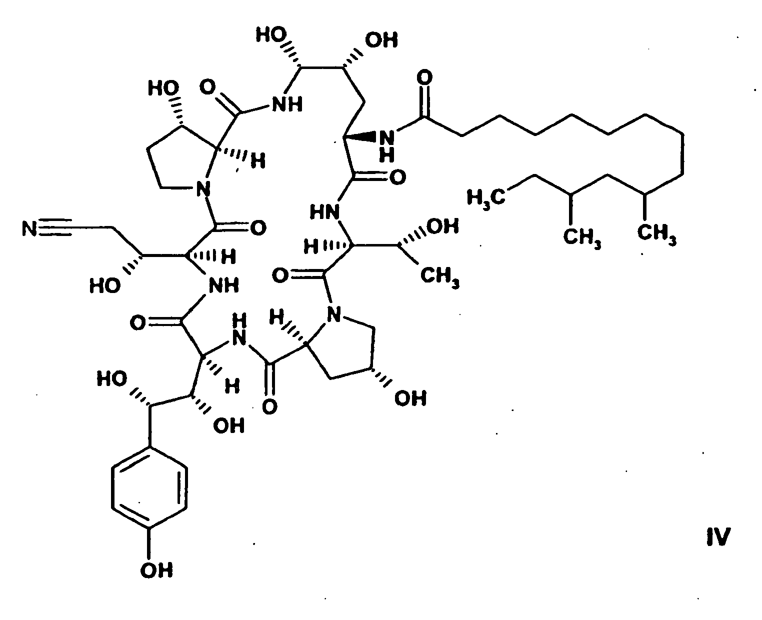
b) reacting the compound of formula IV as obtained in step a) with a thiophenol to obtain a compound of formula V 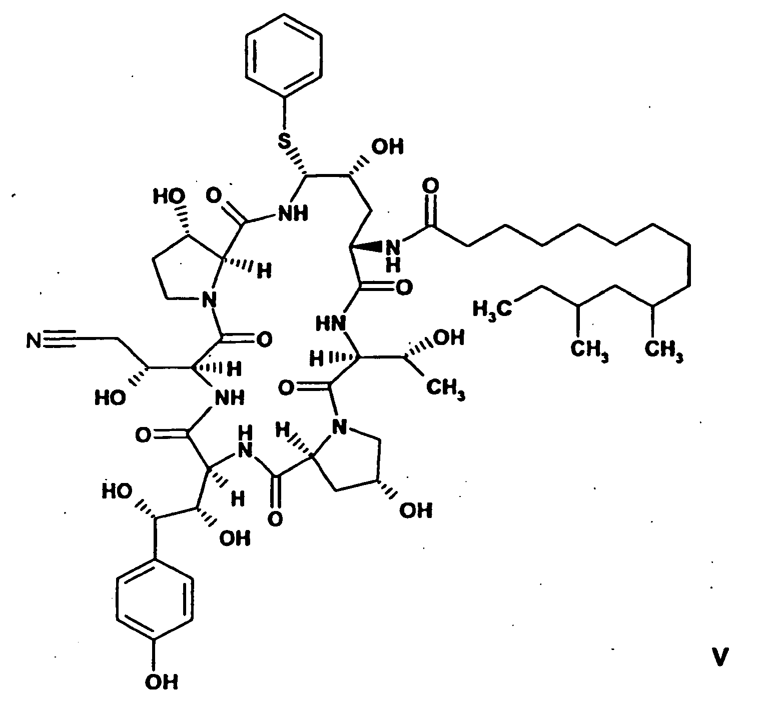
c) reacting the compound of formula V as obtained in step b) with H₂N-CH₂-CH₂-NH₂ to obtain a compound of formula VI or an acid addition salt or a solvate thereof, and
d) optionally isolating the compound of formula VI or an acid addition salt or a solvate thereof as obtained in step c).

Furthermore, the present invention provides the use of a compound of formula II or VI or VIa for preparing caspofungin.

### Detailed Description of the Invention

In preferred embodiments of the above described processes for the production of aza cyclohexapeptide compounds of formula I or of a pharmaceutically acceptable salt thereof R₁ is H and R₂ is selected from H, C₁-C₈ alkyl, C₃-C₄alkenyl, (CH₂)₂₋₄OH, (CH₂)₂₋₄NR₃R₄; or R₁ is C₁-C₈ alkyl and R₂ is selected from H, C₁-C₈ alkyl, C₃-C₄ alkenyl, (CH₂)₂₋₄OH, (CH₂)₂₋₄NR₃R₄; or
R₁ is C₃-C₄ alkenyl and R₂ is selected from H, C₁-C₈ alkyl,C₃-C₄ alkenyl, (CH₂)₂₋₄OH, (CH₂)₂₋₄NR₃R_{4;} or
R₁ is (CH₂)₂₋₄OH and R₂ is selected from H, C₁-C₈ alkyl, C₃-C₄ alkenyl, (CH₂)₂₋₄OH, (CH₂)₂₋₄NR₃R₄; or
R₁ is (CH₂)₂₋₄NR₃R₄ and R₂ is selected from H, C₁-C₈ alkyl, C₃-C₄ alkenyl, (CH₂)₂₋₄OH, (CH₂)₂₋₄NR₃R₄.

In a further preferred embodiment of the above process X is HN-CH₂-CH₂-NH₂.

In still a further preferred embodiment of the above processes aza cyclohexapeptide compounds of formula I or a pharmaceutically acceptable salt thereof are produced wherein X is NR₁R₂ and wherein
R₁ is H, C₁-C₄ alkyl , C₃-C₄ alkenyl, (CH₂)₂₋₄OH or (CH₂)₂₋₄NR₃R₄;
R₂ is H, C₁-C₄ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)_{5,} (CH₂)₂O(CH₂)₂ or (CH₂)₂NH(CH₂)₂;
R₃ is H or C₁-C₄ alkyl;
R₄ is H or C₁-C₄ alkyl.

In additionally preferred embodiments of the above processes for the production of aza cyclohexapeptide compounds of formula I or of a pharmaceutically acceptable salt thereof R₁ is H and R₂ is selected from H, C₁-C₄ alkyl, C₃-C₄ alkenyl, (CH₂)₂₋₄OH, (CH₂)₂₋₄NR₃R₄; or R₁ is C₁-C₄ alkyl and R₂ is selected from H, C₁-C₄ alkyl, C₃-C₄ alkenyl, (CH₂)₂₋₄OH, (CH₂)₂₋₄NR₃R₄; or
R₁ is C₃-C₄ alkenyl and R₂ is selected from H, C₁-C₄ alkyl, C₃-C₄ alkenyl, (CH₂)₂₋₄OH, (CH₂)₂₋₄NR₃R₄; or
R₁ is (CH₂)₂₋₄OH and R₂ is selected from H, C₁-C₄ alkyl, C₃-C₄ alkenyl, (CH₂)₂₋₄OH, (CH₂)₂₋₄NR₃R₄; or
R₁ is (CH₂)₂₋₄NR₃R₄ and R₂ is selected from H, C₁-C₄ alkyl,C₃-C₄ alkenyl, (CH₂)₂₋₄OH, (CH₂)₂₋₄NR₃R₄.

The below described conditions relate to the first process of the invention as described above for the production of aza cyclohexapeptide compounds of formula I or of a pharmaceutically acceptable salt thereof which comprises the steps of
a) reducing a compound of formula II or an acid addition salt thereof wherein X is defined as above, to obtain a compound of formula I or a pharmaceutically acceptable salt thereof, and
b) optionally isolating the compound of formula I or a pharmaceutically acceptable salt thereof as obtained in step a).

Optionally, X may be protected in order to achieve the desired regioselectivity , e.g. if X contains several reactive nucleophilic groups, by protective groups such as amine or hydroxyl protecting groups. Said protective groups may be removed before, simultaneously or after the reduction step.

The reduction of compounds of formula II or acid addition salts thereof in step a) to obtain compounds of formula I may be performed by using any nitrile reducing agent. Preferably, catalytic hydrogenation is applied. Catalysts suitable for the reduction are e.g. catalysts originating from noble metals e.g. palladium, platinum, rhodium, ruthenium, e.g. HRh(PR₃)₃ wherein R represents an optionally substituted phenylgroup or an alkylgroup, e.g. isopropyl, Rh/Al₂O₃, CIR(PR₃)₃, Pt, PtO₂, Pd, Pd on carbon or Ru or RuCl₂ or catalyst such as nickel. Preferably, Rh/Al₂O₃ or Pd on carbon is used as catalyst. The hydrogen source may be H₂ or generated in situ e.g. from ammonium formiate or diimine reactions. Preferably, H₂ is used.

The amount of catalyst used in the reduction step may vary from 5% to 500% weight based on the compound of formula II.

Applied pressures may vary from atmospheric pressure up to 20 bar, e.g. from atmospheric pressure up to 10 bar, e.g. may be up to 5 bar, e.g. up to 3 bar, e.g. may be about 1 bar.

The reduction step may be performed by dissolving or suspending compounds of formula II or acid addition salts thereof in a suitable solvent. Suitable solvents are solvents which are inert to reduction. Such solvents may be identified by a skilled person in routine tests. Suitable solvents are e.g. alcohols such as C₁-C₄ alcohols, e.g. methanol, ethanol or isopropanole, amides such as N,N-Dimethylformamide or n-methylpyrrolidon, optionally in combination with water. A preferred solvent is a mixture of isopropanol and water.

Preferably, the amount of water is more than 5% of the amount of isopropanol. Optionally an acid e.g. acetic acid may be present in the reduction step to suppress impurity formation.

After the reduction step is complete the product may be isolated in step b) by methods known in the art. For instance, the catalyst may be filtered off and the product may be purified by chromatography, e.g. reversed phase chromatography (e.g. a RP-8 or RP-18 type modified silica gel). Isolation from the fractions may be done by lyophilisation and the products may be isolated as free amines or as their addition salts with organic acids such as e.g. formic, acetic, tartaric or trifluoroacetic acid. Caspofungin is preferably isolated as its diacetate.

Surprisingly, the reduction of the nitrile is of high selectivity in the presence of the other functionalities, e.g. in the presence of the aminal moiety present in the aza cyclohexapeptide compounds. Without wishing to be bound by theory, the inventors believe that this high selectivity is one reason for the high yields obtained in the reduction process of the invention, wherein reaction yields may be up to about 80% to about 90%, such as about 82% by weight by applying known HPLC methods and calculating against an external standard. Additionally, in the reduction process of the invention less than 5% starting material are left, e.g. less than 3%, preferably less than 2%.

These high yields of the reduction reaction of the invention are surprising, because prior art processes for reduction of the amide group to the amine group of aza cyclohexapeptide compounds as herein discussed show considerably lower reaction yields such as 47 % (assay) as seen e.g. in US 5,552,521, or such as 61 % (HPLC assay) despite the use of protecting groups as seen in US 5,936,062. Furthermore, more starting material seems to remain after completion of the reduction reactions of prior art as seen e.g. in US 5,552,521 which reports in example 1 a) a ratio of starting material to product of 1:1, and e.g. in US 5,936,062 which discloses in example 2 b) less than 30% starting material remaining at the end of the reaction stage.

Therefore, in a further aspect, the present invention relates to a process of reducing a nitrile group to an amino group in a compound further containing an aminal moiety at the "C5-orn" position by catalytic hydrogenation. The term "C5-orn" is understood to mean the 5-carbon of the 4-hydroxy ornithine component. In particluar, said compound is an aza cyclohexapeptide compound of formula II or a pharmaceutically acceptable salt, e.g. an addition salt, such as an acid addition salt, or a solvate thereof. Preferably, said compound is a compound of formula VI, more preferably a compound of formula VIa, or its salt, e.g. addition salt, or its solvate as described below. The product obtained by said process is an aza cyclohexapeptide compound of formula I or a pharmaceutically acceptable salt thereof. Preferably, said compound is caspofungin.

Thus, the present invention also relates to the use of catalytic hydrogenation for the conversion of a nitrile group to an amino group in a compound further containing an aminal moiety. The compounds mentioned above are preferred compounds for this use.

In addition, the isolation and crystallization of the compound of formula II and/or of a compound of formula VI and/or VIa as herein described allow an easy removal of the epimeric compound at the benzylic position C35 (homotyrosine moiety) amongst the removal of other impurities.

As starting material for said process Pneumocandin B₀ may be used. Pneumocandin B₀ may be further processed by dehydration, reaction with a thiophenol and substitution of the thiophenyl group to obtain a compound of formula II.

Therefore, the present invention also relates to a process for the production of aza cyclohexapeptide compounds of formula I 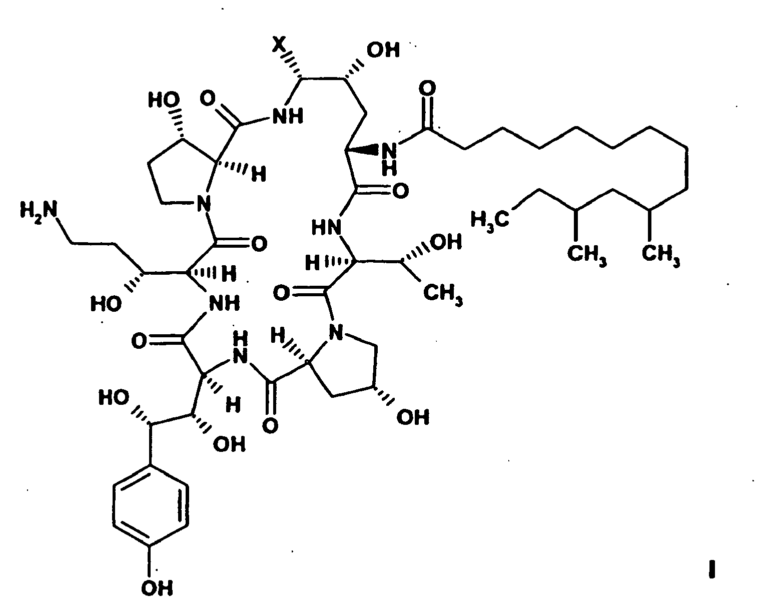 wherein X is NR₁R₂ and wherein
R₁ is H, C₁-C₈ alkyl, C₃-C₄ alkenyl, (CH₂)₂₋₄OH or (CH₂)₂₋₄NR₃R₄;
R₂ is H, C₁-C₈ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₈ alkyl;
R₄ is H or C₁-C₈ alkyl;
or a pharmaceutically acceptable salt thereof comprising the steps of
a) reacting a compound of formula III 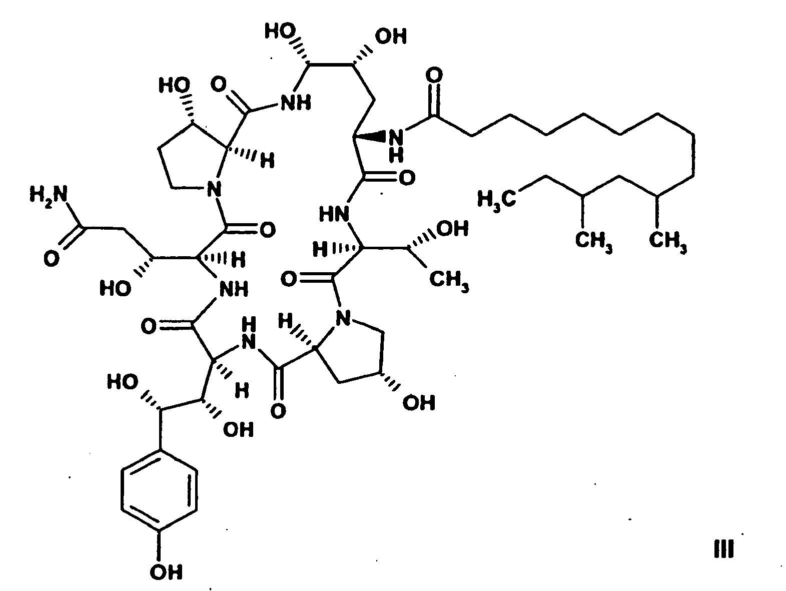 with a dehydrating agent to obtain a compound of formula IV 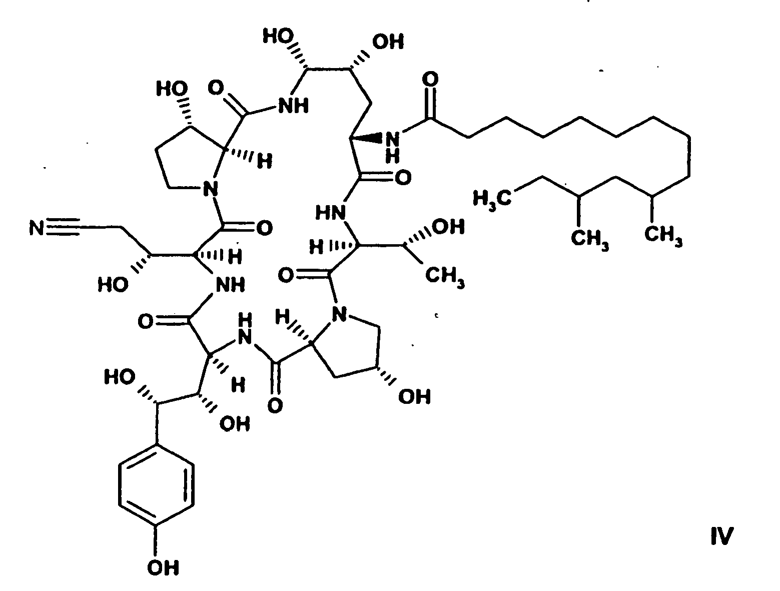
b) reacting the compound of formula IV as obtained in step a) with a thiophenol to obtain a compound of formula V 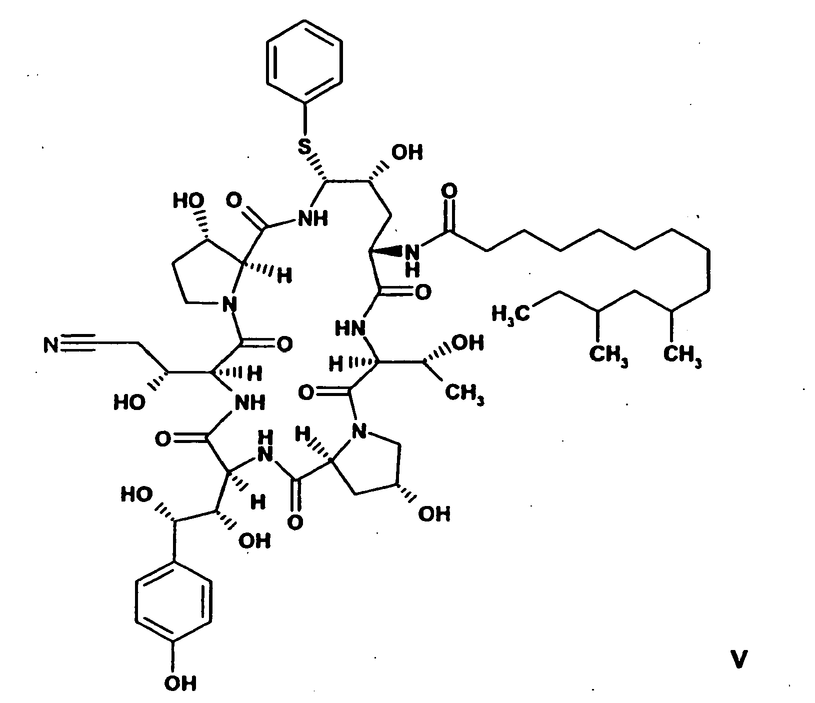
c) reacting the compound of formula V as obtained in step b) with a compound of formula HX, wherein X is as defined above, to obtain a compound of formula II or an acid addition salt thereof,
d) reducing a compound of formula II or an acid addition salt thereof as obtained in step c) to obtain a compound of formula I or a pharmaceutically acceptable salt thereof, and
e) optionally isolating the compound of formula I or a pharmaceutically acceptable salt thereof as obtained in step d).

In preferred embodiments of the above process
R₁ is H and R₂ is selected from H, C₁-C₈ alkyl, C₃-C₄ alkenyl, (CH₂)₂₋₄OH, (CH₂)₂₋₄NR₃R₄; or
R₁ is C₁-C₈ alkyl and R₂ is selected from H, C₁-C₈ alkyl,C₃-C₄ alkenyl, (CH₂)₂₋₄OH, (CH₂)₂₋₄NR₃R₄; or
R₁ is C₃-C₄ alkenyl and R₂ is selected from H, C₁-C₈ alkyl, C₃-C₄ alkenyl, (CH₂)₂₋₄OH, (CH₂)₂₋₄NR₃R₄; or
R₁ is (CH₂)₂₋₄OH and R₂ is selected from H, C₁-C₈ alkyl,C₃-C₄ alkenyl, (CH₂)₂₋₄OH, (CH₂)₂₋₄NR₃R₄; or
R₁ is (CH₂)₂₋₄NR₃R₄ and R₂ is selected from H, C₁-C₈ alkyl, C₃-C₄ alkenyl, (CH₂)₂₋₄OH, (CH₂)₂₋₄NR₃R₄.

In a further preferred embodiment of the above process X is HN-CH₂-CH₂-NH₂.

In still another preferred embodiment of the above process aza cyclohexapeptide compounds of formula I or a pharmaceutically acceptable salt thereof are produced wherein X is NR₁R₂ and wherein
R₁ is H, C₁-C₄ alkyl, C₃-C₄ alkenyl, (CH₂)₂₋₄OH or (CH₂)₂₋₄NR₃R₄;
R₂ is H, C₁-C₄ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₄ alkyl;
R₄ is H or C₁-C₄ alkyl.

In additionally preferred embodiments of the above process
R₁ is H and R₂ is selected from H, C₁-C₄ alkyl, C₃-C₄ alkenyl, (CH₂)₂₋₄OH, (CH₂)₂₋₄NR₃R₄; or R₁ is C₁-C₄ alkyl and R₂ is selected from H, C₁-C₄ alkyl, C₃-C₄alkenyl, (CH₂)₂OH, (CH₂)₂₋₄NR₃R_{4;} or
R₁ is C₃-C₄ alkenyl and R₂ is selected from H, C₁-C₄ alkyl, C₃-C₄ alkenyl, (CH₂)₂₋₄OH, (CH₂)₂₋₄NR₃R₄; or
R₁ is (CH₂)₂₋₄OH and R₂ is selected from H, C₁-C₄ alkyl,C₃-C₄ alkenyl, (CH₂)₂₋₄OH, (CH₂)₂₋₄NR₃R₄; or
R₁ is (CH₂)₂₋₄NR₃R₄ and R₂ is selected from H, C₁-C₄ alkyl, C₃-C₄ alkenyl, (CH₂)₂₋₄OH, (CH₂)₂₋₄NR₃R₄.

The below described conditions relate to the above described further process of the invention for the production of aza cyclohexapeptide compounds of formula I or of a pharmaceutically acceptable salt thereof which comprises the steps
a) reacting a compound of formula III with a dehydrating agent to obtain a compound of formula IV,
b) reacting the compound of formula IV as obtained in step a) with a thiophenol to obtain a compound of formula V,
c) reacting the compound of formula V as obtained in step b) with a compound of formula HX, wherein X is as defined above, to obtain a compound of formula II or an acid addition salt thereof,
d) reducing a compound of formula II or an acid addition salt thereof as obtained in step c) to obtain a compound of formula I or a pharmaceutically acceptable salt thereof, and
e) optionally isolating the compound of formula I or a pharmaceutically acceptable salt thereof as obtained in step d).

Step a), i.e. reacting a compound of formula III with a dehydrating agent to obtain a compound of formula IV may be carried out according to methods known in the art, e.g. under conditions known for such type of reaction, for instance as described in EP 535967 A. Suitable reagents to dehydrate the amide of formula III are anhydrides such as acetic anhydride, trifluoroacetic anhydride and phosphorus pentoxide, acid chlorides such as oxalyl chloride, phosphorus oxychloride etc., phosphonium reagents such as triphenylphosphoniumchloride, carbodiimides such as dicyclohexylcarbodiimide or other dehydrating agents such as cyanuric chloride, aluminium chloride or titanium tetrachloride. Preferably, cyanuric chloride as described e.g. in EP 535967 A is used.

Step b), i.e. reacting the compound of formula IV with a thiophenol to obtain a compound of formula V may be carried out in analogy, e.g. according to methods known in the art, for example as disclosed in EP 912603 and/or in WO 97/47645. Accordingly, a compound of formula IV may be reacted with a thiophenol in acetonitrile and trifluoroacetic acid to produce a thiophenol containing intermediate of formula V. Any moderate strength acid, e.g. trifluoroacetic acid, phosphoric acid or trichloroacetic acid is expected to yield the intermediate of formula V in good yield. Other mercaptans may also be used such as substituted thiophenols, e.g. thiophenols substituted by one or more halogen, e.g. fluoro, chloro, bromo or iodo, C₁₋₄ alkyl, C₁-C₄ alkoxy, e.g. such as 4-methoxythiophenol, or such as substituted heterocycles, e.g. 2-mercapto-1-methylimidazole, 2-mercaptobenzthiazole, and the like. Preferably, thiophenol is used.

Step c), i.e. substituting the thiophenol of a compound of formula V with a nucleophilic compound of formula HX wherein X is defined as herein described may be carried out in analogy, e.g. according to methods known in the art, e.g. the thiophenyl group may be displaced e.g. by an amino group by reaction with the corresponding amine, preferably by displacement with neat ethylendiamine. Step c) may also be carried out e.g. in analogy, e.g. according to methods as disclosed in WO 97/47645.

Step d), i.e. reducing a compound of formula II or an acid addition salt thereof to obtain a compound of formula I or a pharmaceutically acceptable salt thereof may be carried out as described above. Step d) may e.g. be carried out as described in claim 4, and/or may be carried out as described above for the first process of the invention by applying the conditions therefore mentioned.

Step e), i.e. isolating a compound of formula I or a pharmaceutically acceptable salt thereof may be carried out according to methods known in the art or as herein described.

Optionally, X may be protected in order to achieve the desired regioselectivity , e.g. if X contains several reactive nucleophilic groups, by protective groups such as amine or hydroxyl protecting groups. Said protective groups may be removed before, simultaneously or after the reduction step.

Preferably, in step c) as described above the compound of formula HX is ethylendiamine leading to caspofungin after reduction according to step d) as described above.

Preferably, the compound of formula I obtained in the herein described processes is caspofungin having the formula Ia, and its pharmaceutically acceptable salts: 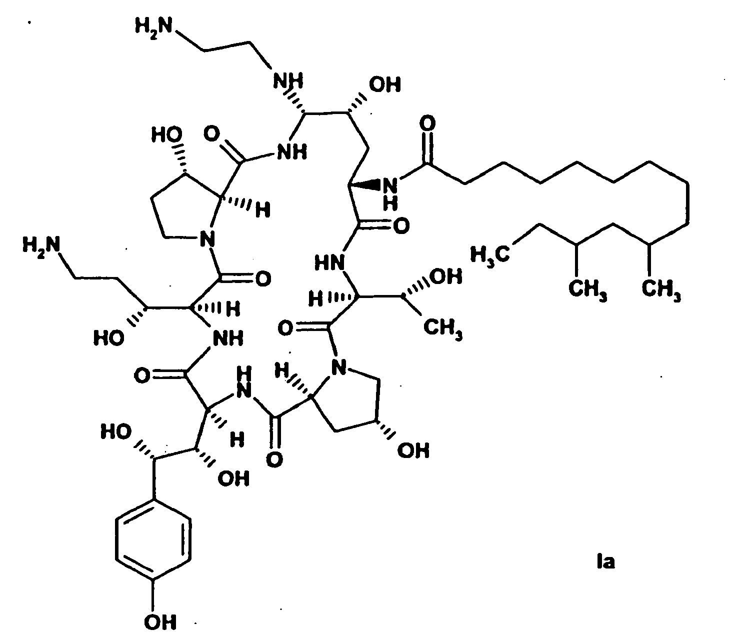 Compounds of formula II and the addition salts, e.g. acid addition salts, and solvates thereof are novel. Accordingly, in another aspect the present invention relates to a compound of formula II 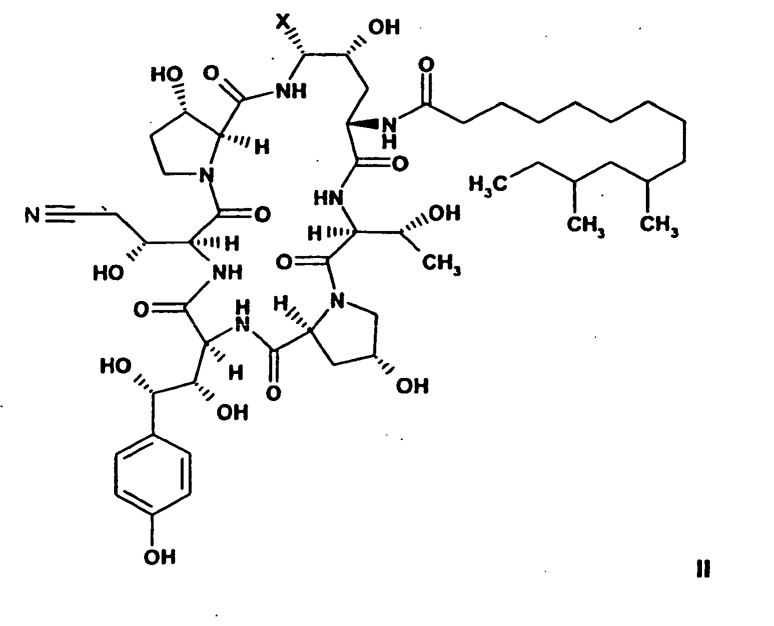 or an acid addition salt or a solvate thereof, wherein X is NR₁R₂, and wherein
R₁ is H, C₁-C₈ alkyl, C₃-C₄ alkenyl, (CH₂)₂₋₄OH or (CH₂)₂₋₄NR₃R₄;
R₂ is H, C₁-C₈ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₈ alkyl;
R₄ is H or C₁-C₈ alkyl.
One preferred embodiment of a compound of formula II is a compound of formula VI or an acid addition salt or a solvate thereof, such as e.g. a compound of formula VIa as described below.

Acid addition salts of compound II and/or VI may be addition salts with a mineral acid such as e.g. hydrochloric acid or with an organic acid such as e.g. formic acid, acetic acid or trifluoroacetic acid. Compounds of formula II or VI may also exist as free amines.

In another aspect the present invention relates to a compound of formula VI 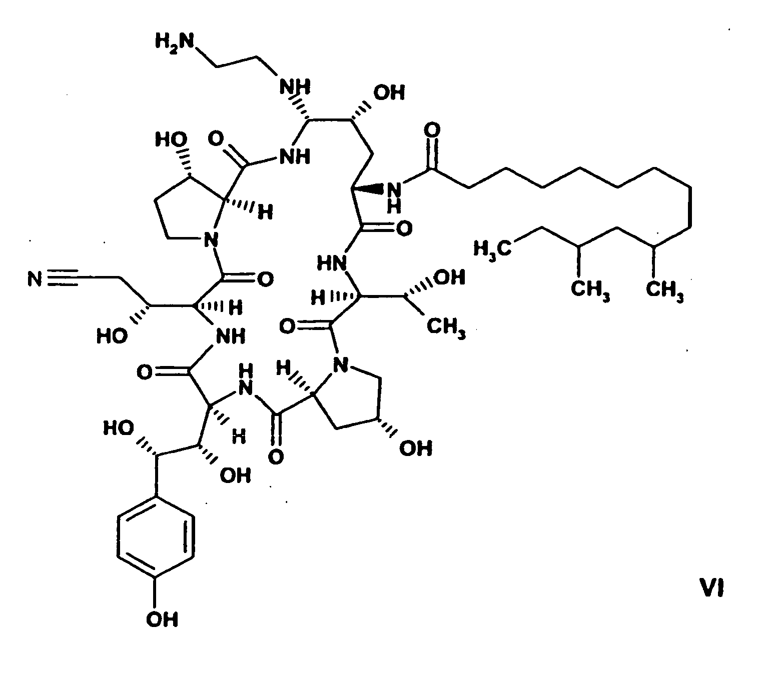 or an acid addition salt or a solvate thereof. This compound is a valuable intermediate in the preparation of caspofungin due to its enhanced stability as it may be obtained in crystalline form as a monoaddition salt with acetic acid. Accordingly, the monoacetate of the compound of formula VI, i.e. compound of formula VIa is a preferred embodiment of the present invention:

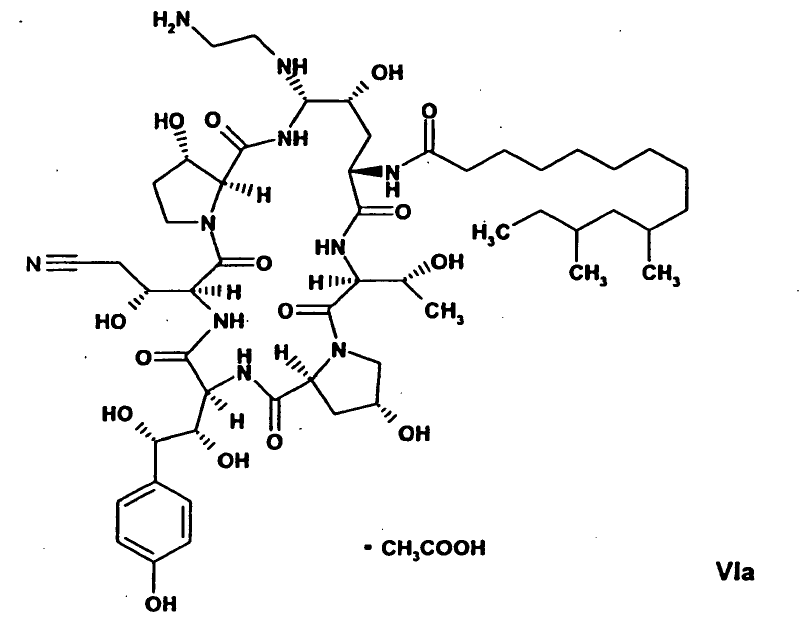

The compound of formula VI may be isolated from the reaction mixture by chromatography on e.g. RP-18 material followed by lyophilisation of rich cut fractions. For chromatography a mixture of acetic acid and acetonitrile may be used wherein the ratio of acetic acid to acetonitrile is from about 60 to 40 to about 80 to 20, e.g. is about 70 to 30, such as 75 to 25. The amorphous compound of formula VI may be then dissolved in an organic solvent , e.g. in an alcohol such as e.g. methanol or ethanol in the presence of acetic acid. The monoacetate of a compound of formula VI may be crystallized by the addition of an antisolvent such as an ester, e.g. an acetic acid C₁-C₄ alkylester such as e.g. ethylacetate. In addition, impurities such as the epimer at C35 (homotyrosin moiety) are removed efficiently by the crystallization process, impurities which are difficult or almost impossible to be removed by chromatography in an economic way.

Compounds of formula II are intermediates useful for the preparation of aza cyclopeptide compounds as herein described. In particular, compounds of formulae VI and VIa are valuable intermediates for the preparation of caspofungin.

Accordingly, in a further aspect the present invention relates to the use of a compound of formula II or of an addition salt or a solvate thereof in the preparation of caspofungin.

Additionally, a still further aspect the present invention relates to the use of a compound of formula VI or an addition salt or a solvate thereof, or of its monoacetate, i.e. a compound of formula VIa, in the preparation of caspofungin. The use of said compounds advantageously leads to high reaction yields of the reduction of the nitrile group of a compound of formula II , to an amine as obtained in a compound of formula I as herein described. Said reaction yields of the reduction process of the invention are up to about 80% to about 90% and thus considerably higher than those observed in prior art processes involving other aza cyclopeptide compounds e.g. as reported in EP 535967 A where the reaction yield is about 43%.

In a further aspect, the present invention relates to a process for the production of a compound of formula II or an acid addition salt or a solvate thereof comprising the steps of
a) reacting a compound of formula III as described above with a dehydrating agent to obtain a compound of formula IV as described above,
b) reacting the compound of formula IV as obtained in step a) with a thiophenol to obtain a compound of formula V as described above,
c) reacting the compound of formula V as obtained in step b) with a compound of formula HX, wherein X is NR₁R₂, and wherein X, R₁ and R₂ are as herein described, to obtain a compound of formula II or an acid addition salt or a solvate thereof, and
d) optionally isolating the compound of formula II or an acid addition salt or a solvate thereof as obtained in step c).

In a further embodiment, the present invention relates to a process for the production of a compound of formula VI or an acid addition salt or a solvate thereof comprising the steps of
a) reacting a compound of formula III as described above with a dehydrating agent to obtain a compound of formula IV as described above,
b) reacting the compound of formula IV as obtained in step a) with a thiophenol to obtain a compound of formula V as described above,
c) reacting the compound of formula V as obtained in step b) with H₂N-CH₂-CH₂-NH₂ to obtain a compound of formula VI or an acid addition salt or a solvate thereof, and
d) optionally isolating the compound of formula VI or an acid addition salt or a solvate thereof as obtained in step c).

The dehydrating agent suitable for use in step a) of the above described processes is as defined above. Preferably, the dehydrating agent is cyanuric chloride.

Preferably, the compound of formula VI is isolated in step d) above as its monoacetate salt, i.e. as compound of formula Via.

### Examples

The following Examples will illustrate the present invention but are not intended to limit the present invention in any way. All temperatures are given in degree Celsius and are uncorrected.

### Example 1: Preparation of the compound of formula IV

Pneumocandin B₀ (10.2 g) is dissolved in a mixture of dry 1-methyl-2-pyrrolidon (90 ml) and dry N,N-dimethylformamide (10 ml). The pale yellow solution is chilled to -20°C and cyanuric chloride (4.2 g) is added in one portion. The mixture is stirred at -20°C until a 98% conversion is reached (HPLC, approx. 3.5 hours). Water (100 ml) is added over 10 minutes, and the mixture is warmed to ambient temperature.
The crude mixture is slowly poured into vigorously stirred water (1400 ml). The suspension is aged for 2 hours and then filtered. The product is thoroughly washed with water and then dried under vacuum. This material (9.3 g) is used in the next step without further purification.

### Example 2: Preparation of the compound of formula IV

Pneumocandin B₀ (10.0 g) is dissolved in dry N,N-dimethylformamide (100 ml). The water content of the solution is determined and is adjusted to ca. 0.15%. The solution is chilled to -20°C and cyanuric chloride (4.2 g) is added in one portion. The mixture is stirred at - 20°C until a 97 % conversion is reached (HPLC, approx. 1.0 hour). Water (100 ml) is added over 10 minutes, and the mixture is warmed to ambient temperature.

The crude mixture is slowly poured into vigorously stirred water (1400 ml). The suspension is aged for 2 hours and then filtered. The product is thoroughly washed with water and then dried under vacuum. This material (8.2 g) is used in the next step without further purification.

### Example 3: Preparation of Caspofungin

The compound of formula VI (500 mg) is dissolved in a 9:1 mixture of 2-propanol/water (13 ml) and acetic acid (650 µl). The mixture is treated with activated charcoal (50 mg) and filtered. To the filtrate is added ammonium acetate (1.35 g) and Rh/Al₂O₃ (5 % Rhodium, 105 mg). The resulting mixture is treated with hydrogen (atmospheric pressure) at room temperature for approximately 24 hours. Activated carbon (100 mg) is added and the mixture is filtered. The filtrate is evaporated under reduced pressure. The residue is dissolved in methanol (10 ml) and water (50 ml) and loaded on a preparative C-18 column. The product is eluted with 22 % acetonitril/water (0.15 % acetic acid). The rich cut fractions are pooled and lyophilized to give caspofungin diacetate (291 mg) as an amorphous white solid.

### Example 4: Preparation of Caspofungin

The compound of formula VI (250 mg) is dissolved in a 8:2 mixture of 2-propanol/water (25 ml) and acetic acid (325 µl). The mixture is treated with activated charcoal (25 mg) and filtered. To the filtrate is added Pd/C (10 % Palladium, 250 mg) and ammonium formiate (2.03 g). The resulting mixture is stirred vigorously at room temperature for approximately 24 hours. The mixture is filtered. The filtrate is diluted with water and loaded on a preparative C-18 column. The product is eluted with 22 % acetonitrile/water (0.15 % acetic acid). The rich cut fractions are pooled and lyophilized to give caspofungin diacetate (94 mg) as an amorphous white solid.

### Example 5: Preparation of Caspofungin

The compound of formula VI (20 g) is dissolved in a 85:15 mixture of 2-propanol/water (284 ml) and acetic acid (20 ml). Ammonium acetate (50 g) and Rh/Al₂O₃ (5 % Rhodium, 2 g) are added. The resulting mixture is treated with hydrogen (1 bar) at 30°C until less than 2% of starting material is left and a reaction yield of 82% (by weight*) is reached which takes place within approximately 7.5 hours. Then the catalyst is filtered off. The filtrate is stirred with a metal scavenging agent (e.g. MSA-FC C-1, 17 g) for 2 hours at 30°C and then filtered again. The filter cake is washed with 2-propanol (3 times 20 ml each). The filtrate and washings are combined and evaporated under reduced pressure. The residue is dissolved in methanol (250 ml) and water (1250 ml) and purified by preparative HPLC using a reversed phase C-8 column. The product is eluted with 22 % acetonitrile/water (0.15 % acetic acid). The rich cut fractions are pooled and lyophilized to give caspofungin diacetate (13.7 g) as an amorphous white solid.
* Determined against an external standard using known HPLC method and applying the following conditions:
Apparatus Agilent™ 1100 Series;
Column: Agilent Zorbax™ 300SB-C18, 3.5 µm, 4.6 x 150 mm
Flow rate: 1.5 ml/min: detection: 220 nm; Temperature: 30°C; Injection volume: 20 µl Gradient: Mobile Phase A: 1800 ml H₂O / 200 ml CH₃CN / 1 ml CF₃COOH + Mobile Phase B:100 ml H₂O / 900 ml CH₃CN / 0.5 ml CF₃COOH
Gradient: 0 min: 25% B; 17 min: 50.5% B; 17.1 min: 70% B; 20 min: 70% B; 20.1 min: 25% B; 25 min: 25% B.

The solid (2 g) is dissolved in ethanol (21.7 ml) and water (2.35 ml) at 25°C. Undissolved material is removed by filtration. To the filtrate is added acetic acid (122 µl) and subsequently ethyl acetate (17.5 ml) is added slowly (2 hours). The solution is seeded and stirred for 1 hour at 25°C. Another portion of ethyl acetate (22.5 ml) is added during 5 hours and the crystal suspension is aged for 1 hour. The crystalline solid is filtered off and washed with a mixture of ethanol/water/ethyl acetate (22 ml/2.5 ml/40 ml). The wet cake is dried in a stream of nitrogen to yield 1.7 g of caspofungin diacetate.

### Example 6: Preparation of the compound of formula V

6.0 g of compound of formula IV is suspended in 240 ml dry acetonitrile and cooled to -15°C. 2.6 g thiophenol are added. 12.7 ml trifluoroacetic acid are added at a temperature below -10°C. The reaction mixture is stirred at -15 to -10°C until the content of compound of formula IV is lower than approximately 3% (HPLC) in the reaction mixture. 581 ml cold water are added to the reaction suspension in 1h. The precipitate is isolated and washed with acetonitrile/water 3:1 till the washing solution has a pH above 5.
5.6 g of compound of formula V are obtained. This material is used in the next step without further purification.

### Example 7: Preparation of the compound of formula VI

15.0 g of compound of formula V are added to 42.6 ml neat ethylenediamine and stirred at ambient temperature until the starting material is completely consumed . 72 ml methanol are added at a temperature of below 25°C. Subsequently 204 ml acetic acid (65%) are added at the same temperature. Further 206 ml water are added.
The yellowish solution is extracted with 140 ml n-heptane. The layers are separated and the organic layer is back-extracted using 38 ml acetic acid (65%). The aqueous layers are filtrated and chromatographed using 0.15% acetic acid/ acetonitrile 70/30. The rich cuts are combined and lyophilized. 7.65 g of the compound of formula VI are obtained as acetate adduct.

### Example 8: Crystallization of the compound of formula VI as mono acetate addition salt

69.9 g of amorphous compound of formula VI with an assay of 80% ( HPLC) and a content of the epimer at C35 of 3.0% (HPLC) prepared as described in example 5 are dissolved in 760 ml of methanol and 4.3 ml of glacial acetic acid at ambient temperature. 645 ml of ethyl acetate are added during 1h. The mixture is seeded and stirred for another hour. After addition of another 1.2 l of ethyl acetate during 2h the crystalline product is isolated by filtration. The filter cake is washed with a mixture of 183 ml of methanol, 490 ml of ethyl acetate and 16 ml of water. The product is dried in vacuo yielding 53 g of crystalline compound of formula VI as monoacetate addition salt (yield 88%).
Assay 93.1 % (HPLC)
Water content: 3.0 %
Impurity C35-epimer: 0.1 %
MS (LC-MS, ESI)
1098.7 [M+H]
¹H-NMR (CD₃OD, 300 MHz)
7.15 (d, J=8.5Hz, 2H), 6.78 (d, J=8.5Hz, 2H), 4.55 (m, 5H), 4.3 (m, 5H), 4.19 (d, J=4.5Hz, 1H), 4.1-3.75 (m, 5H), 3.15-2.65 (m, 6H), 2.45 (m, 1H), 2.35-1.85 (m, 8H), 1.93 (s, 3H), 1.7-0.8 (m, 36H)
¹³C-NMR (CD₃OD, 75 MHz)
10.59, 18.5, 19.2, 19.74, 22.39, 22.86, 26.1, 27.01, 29.32, 29.35, 29.54, 29.72, 30.11, 30.21. 31.88, 33.62, 35.98, 37.04, 37.5, 39.04. 42.24, 44.9, 46.02, 49.96, 53.89, 54.92, 56.13, 57.6, 61.65, 63.06, 67.27, 68.31, 68.77, 69.48, 70.33, 74.04, 74.67, 76.21,115.27, 118.71, 128.71, 131.96, 157.46, 167.28, 171.76, 171.88, 172.5, 172.66, 172.85, 175.22, 178.82

### Example 9: Crystallization of the compound of formula VI as mono acetate addition salt

69.9 g of amorphous compound of formula VI with an assay of 80% ( HPLC) and a content of the epimer at the benzylic position C35 (homotyrosine moiety) of 3.0% (HPLC) prepared as described in example 7 are dissolved in 760 ml of methanol and 4.3 ml of glacial acetic acid at ambient temperature. 645 ml of ethyl acetate are added during 1h. The mixture is seeded and stirred for another hour. After addition of another 1.2 l of ethyl acetate during 2h the crystalline product is isolated by filtration. The filter cake is washed with a mixture of 183 ml of methanol, 490 ml of ethyl acetate and 16 ml of water. The product is dried in vacuo yielding 53 g of crystalline compound of formula VI as monoacetate addition salt (yield 88%).
Assay 93.1 % (HPLC, calculated as free base)
Water content: 3.0 %
Impurity C35-epimer: 0.1%
MS (LC-MS, ESI)
1098.7 [M+H]
¹H-NMR (CD₃OD, 300 MHz)
7.15 (d, J=8.5Hz, 2H), 6.78 (d, J=8.5Hz, 2H), 4.55 (m, 5H), 4.3 (m, 5H), 4.19 (d, J=4.5Hz, 1H), 4.1-3.75 (m, 5H), 3.15-2.65 (m, 6H), 2.45 (m, 1H), 2.35-1.85 (m, 8H), 1.93 (s, 3H), 1.7-0.8 (m, 36H)
¹³C-NMR (CD₃OD, 75 MHz)
10.59, 18.5, 19.2, 19.74, 22.39, 22.86, 26.1, 27.01, 29.32, 29.35, 29.54, 29.72, 30.11, 30.21, 31.88, 33.62, 35.98, 37.04, 37.5, 39.04, 42.24, 44.9, 46.02, 49.96, 53.89, 54.92, 56.13, 57.6, 61.65, 63.06, 67.27, 68.31, 68.77, 69.48, 70-33, 74.04, 74.67, 76.21, 115.27, 118.71, 128.71, 131.96, 157.46, 167.28, 171.76, 171.88, 172.5, 172.66, 172.85, 175.22, 178.82
HPLC is performed according to known methods and applying the following conditions:
flow rate: 1.5 ml / min; column temperature: 30°C; Stop time: 8.5 min; Post run: 1.5 min wave length: 220 nm; injection volume: 10µl (microlitre)
Eluent A: 900 ml HPLC-water /100 ml acetonitrile gradient grade /0.5 ml trifluoroacetic acid;
Eluent B: 100 ml HPLC-water / 900 ml acetonitrile gradient grade / 0.5 ml trifluoroacetic acid
Stationary Phase: Agilent^{™} Technologies, Zorbax ^{™} 300 SB-C18, Rapid Resolution, 4.6 mm x 100 mm, 3.5-Micron.
Gradient: 0 min: 37% B; 2 min: 40 % B; 4 min: 46 % B; 7 min: 70 % B; 8.5 min: 70 % B

## Claims

1. A compound of formula II 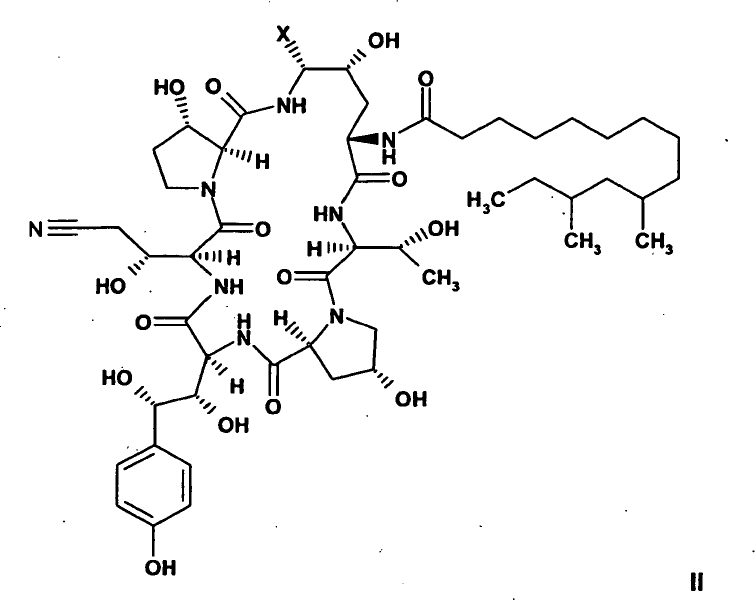 or an acid addition salt or a solvate thereof, wherein X is NR₁R₂, and wherein
R₁ is H, C₁-C₈ alkyl, C₃-C₄ alkenyl, (CH₂)₂₋₄OH or (CH₂)₂₋₄NR₃R₄;
R₂ is H, C₁-C₈ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₈ alkyl;
R₄ is H or C₁-C₈ alkyl.

2. A compound according to claim 1 of formula VI 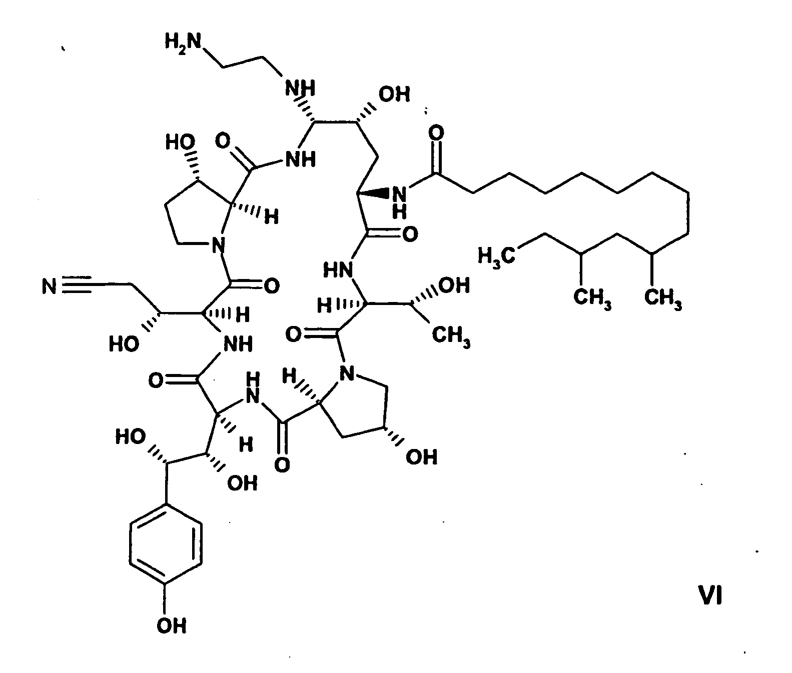 or an acid addition salt or a solvate thereof.

3. A compound according to claim 1 or 2 of formula VIa

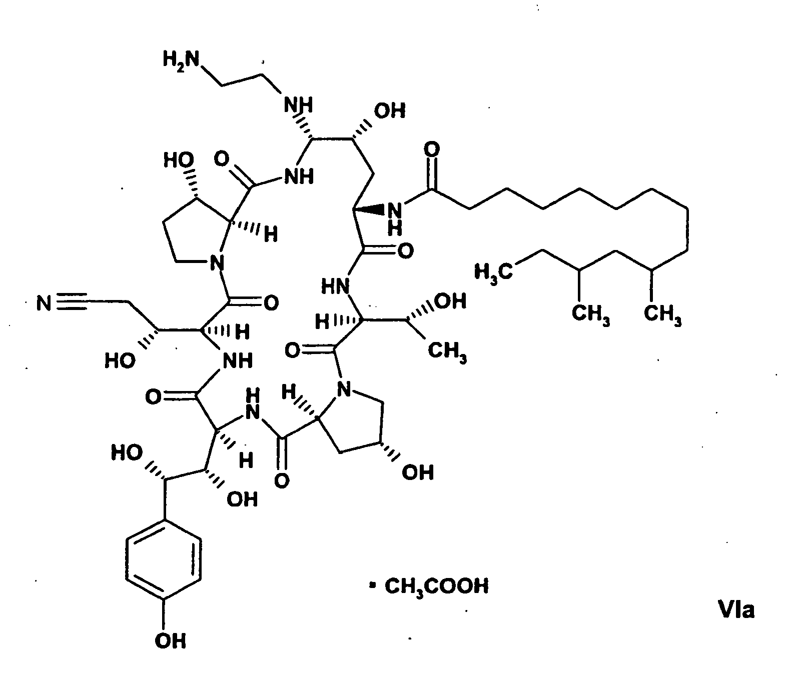

4. A process for the production of aza cyclohexapeptide compounds of formula I 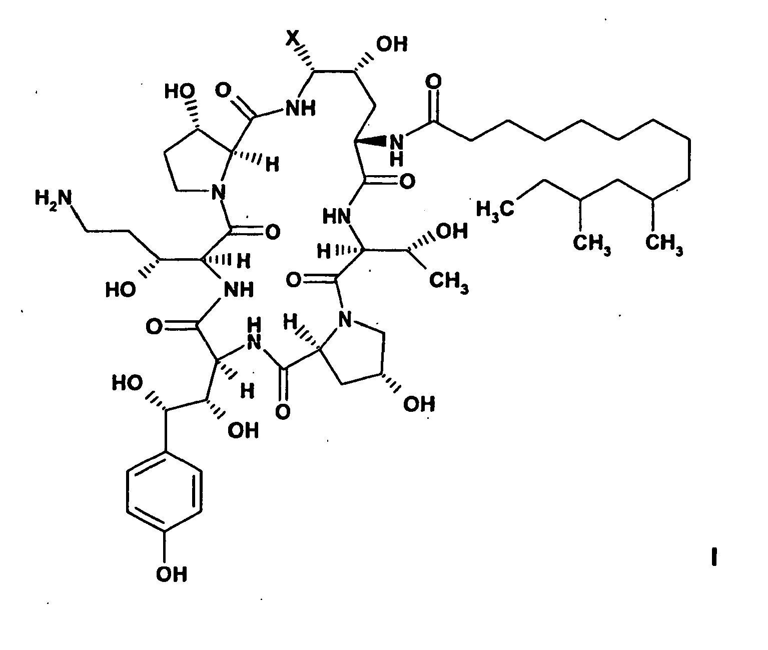 wherein X is NR₁R₂ and wherein
R₁ is H, C₁-C₈ alkyl, C₃-C₄ alkenyl, (CH₂)₂₋₄OH or (CH₂)₂₋₄NR₃R₄;
R₂ is H, C₁-C₈ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₈ alkyl;
R₄ is H or C₁-C₈ alkyl;
or a pharmaceutically acceptable salt thereof comprising the steps of
a) reducing a compound of formula II or an acid addition salt thereof 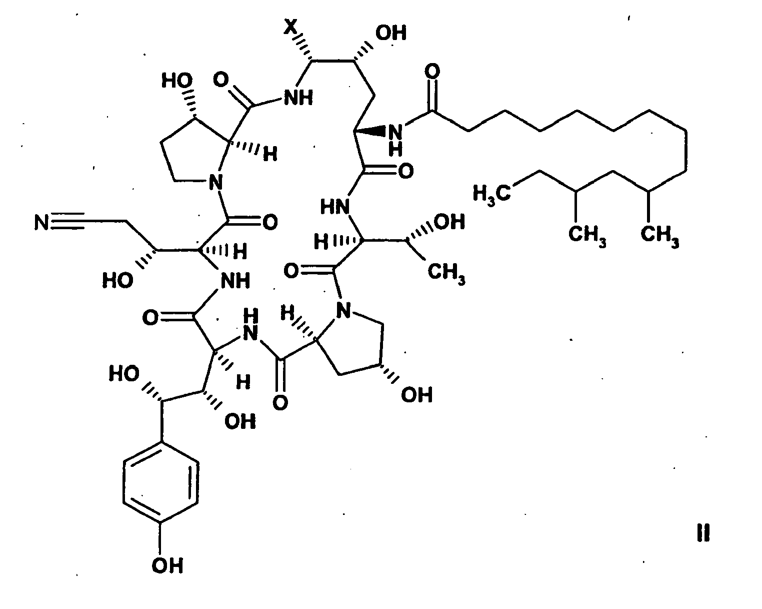 wherein X is defined as above, to obtain a compound of formula I or a pharmaceutically acceptable salt thereof, and
b) optionally isolating the compound of formula I or a pharmaceutically acceptable salt thereof as obtained in step a).

5. A process according to claim 4 for the production of aza cyclohexapeptide compounds of formula I as defined in claim 4
or a pharmaceutically acceptable salt thereof comprising the steps of
a) reacting a compound of formula III 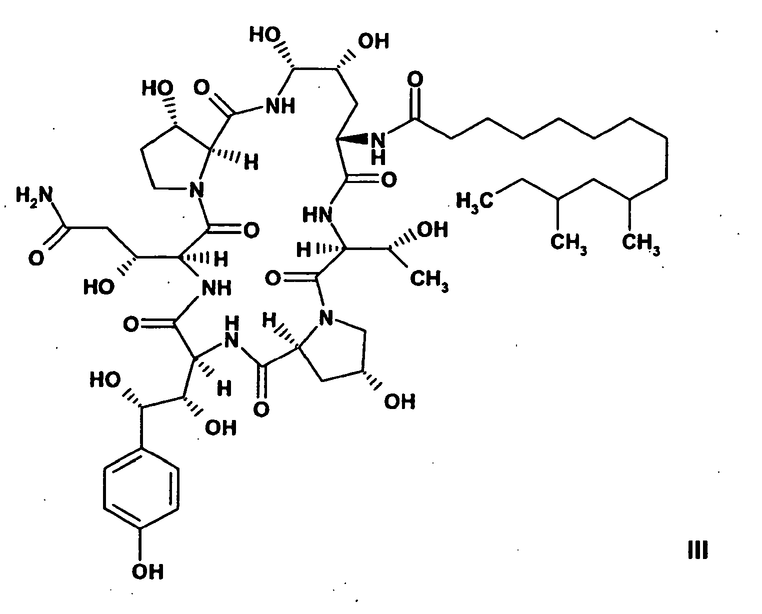 with a dehydrating agent to obtain a compound of formula IV 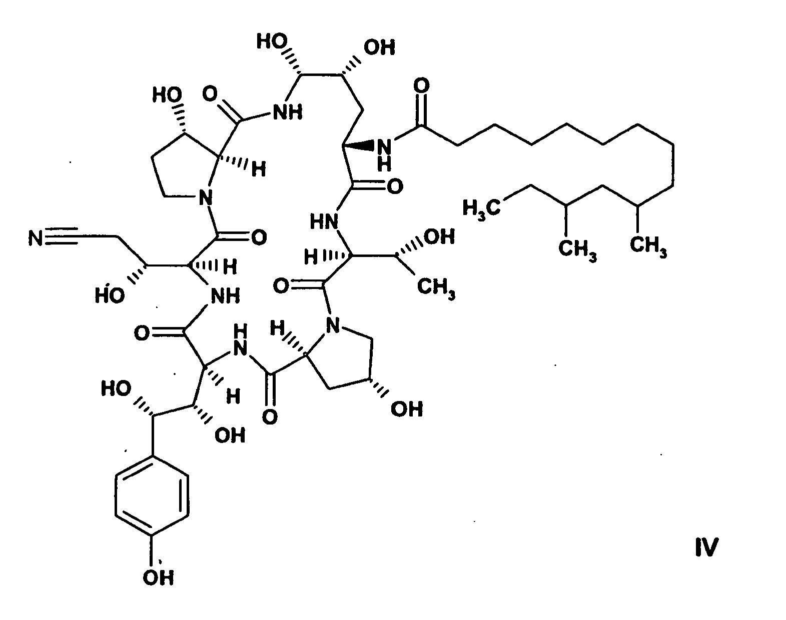
b) reacting the compound of formula IV as obtained in step a) with a thiophenol to obtain a compound of formula V 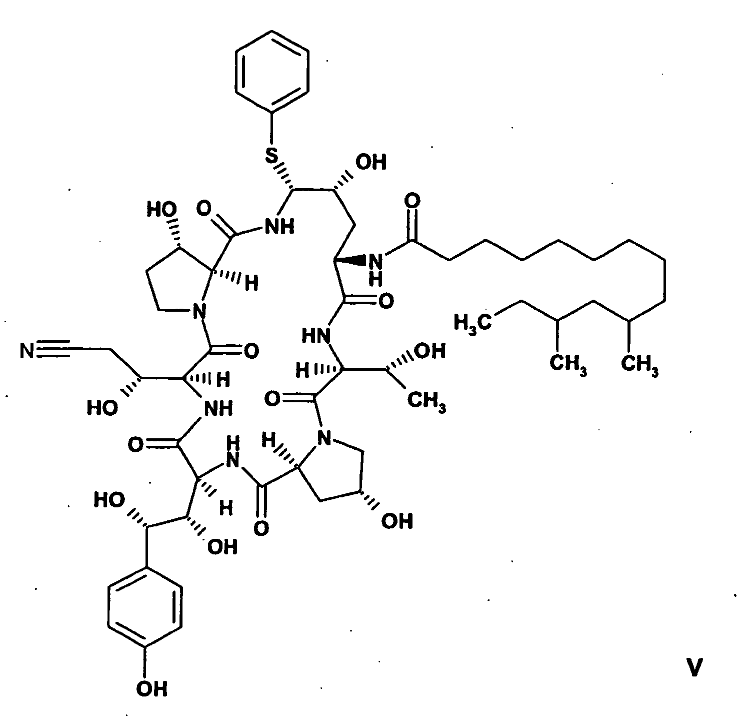
c) reacting the compound of formula V as obtained in step b) with a compound of formula HX, wherein X is as defined in claim 4, to obtain a compound of formula II as defined in claim 1 or an acid addition salt thereof,
d) reducing a compound of formula II or an acid addition salt thereof as obtained in step c) to obtain a compound of formula I or a pharmaceutically acceptable salt thereof, and
e) optionally isolating the compound of formula I or a pharmaceutically acceptable salt thereof as obtained in step d).

6. A process according to claim 4 or 5 wherein X is HN-CH₂-CH₂-NH₂.

7. A process according to claim 4 or 5 wherein the reduction step is carried out by catalytic hydrogenation.

8. A process according to claim 7 wherein Rh/Al₂O₃ or Pd on carbon is used as catalyst.

9. A process according to any one of claims 4 to 8 wherein in the reduction step H₂ is used as hydrogen source.

10. A process for the production of a compound of formula II according to claim 1 or an acid addition salt or a solvate thereof comprising the steps of
a) reacting a compound of formula III 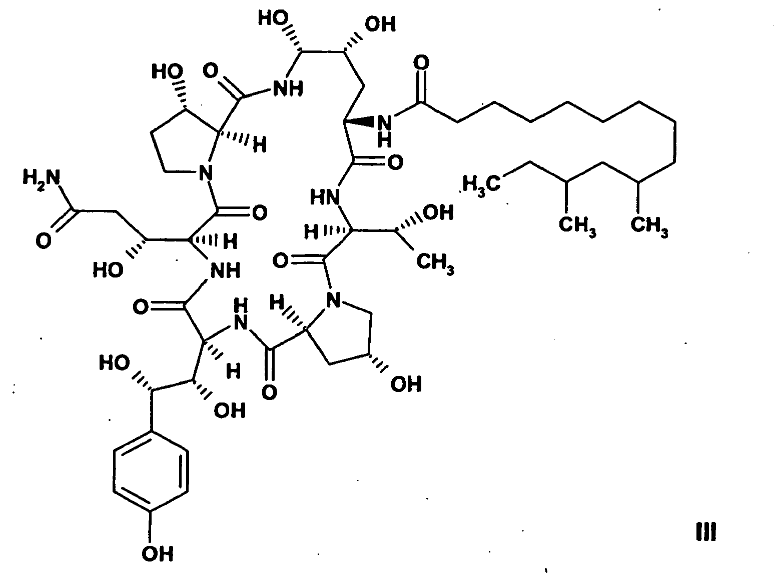 with a dehydrating agent to obtain a compound of formula IV 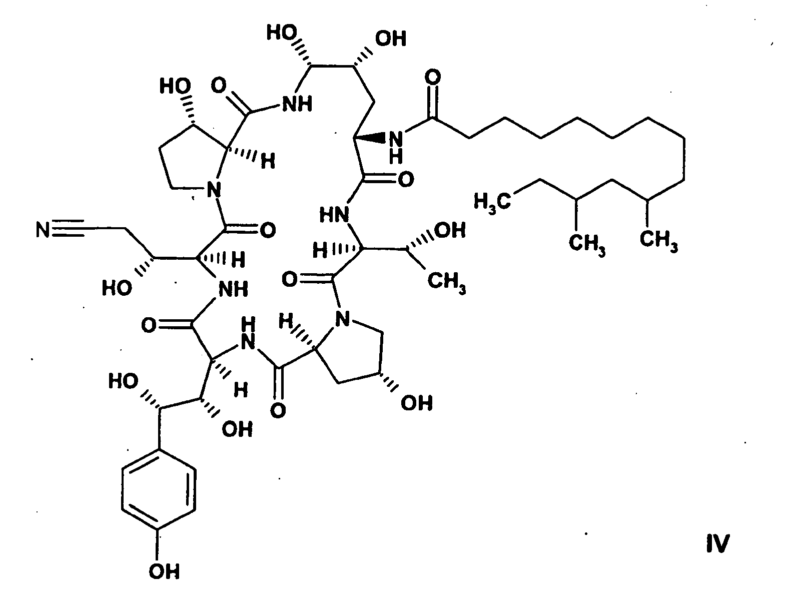
b) reacting the compound of formula IV as obtained in step a) with a thiophenol to obtain a compound of formula V 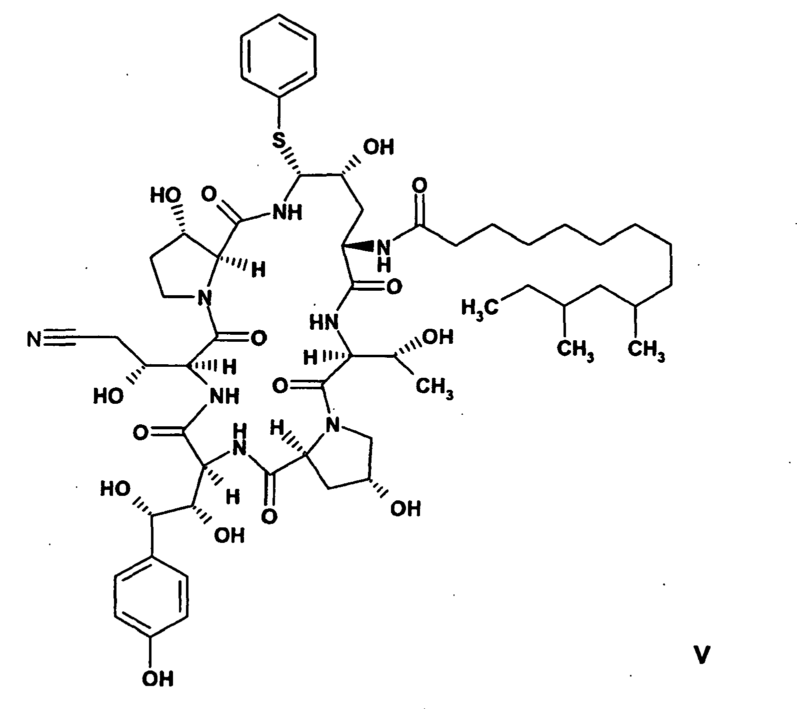
c) reacting the compound of formula V as obtained in step b) with a compound of formula HX, wherein X is NR₁R₂, and wherein
R₁ is H, C₁-C₈ alkyl, C₃-C₄ alkenyl, (CH₂)₂₋₄OH or (CH₂)₂₋₄NR₃R₄;
R₂ is H, C₁-C₈ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₈ alkyl;
R₄ is H or C₁-C₈ alkyl;
to obtain a compound of formula II or an acid addition salt or a solvate thereof, and
d) optionally isolating the compound of formula II or an acid addition salt or a solvate thereof as obtained in step c).

11. A process according to claim 10 for the production of a compound of formula VI according to claim 2 or an acid addition salt or a solvate thereof comprising the steps of
a) reacting a compound of formula III 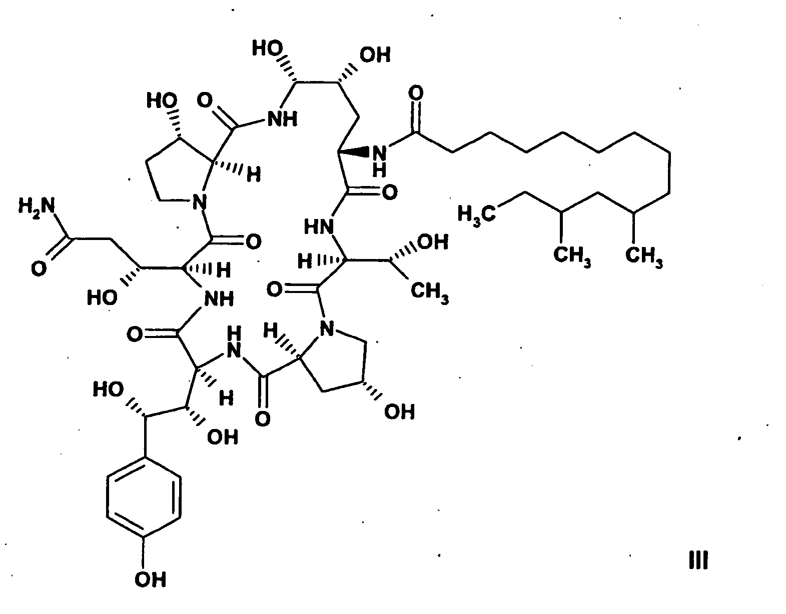 with a dehydrating agent to obtain a compound of formula IV 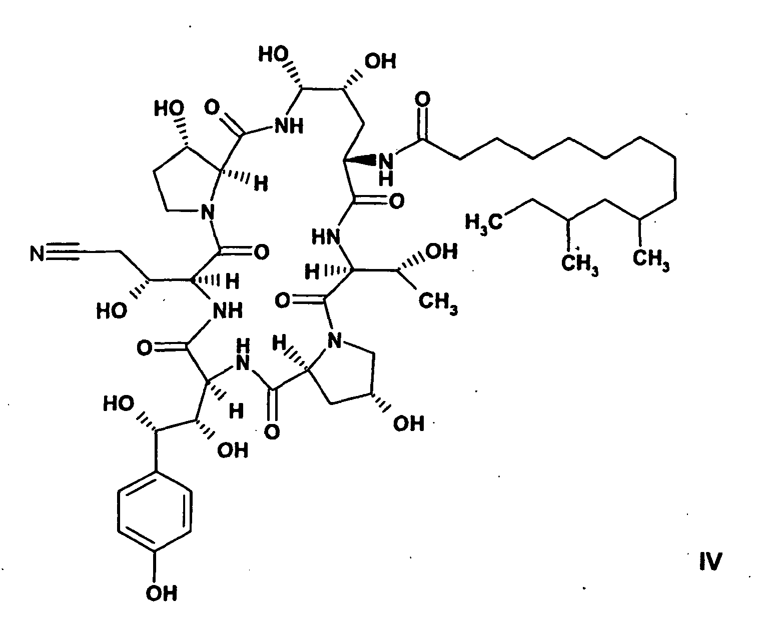
b) reacting the compound of formula IV as obtained in step a) with a thiophenol to obtain a compound of formula V 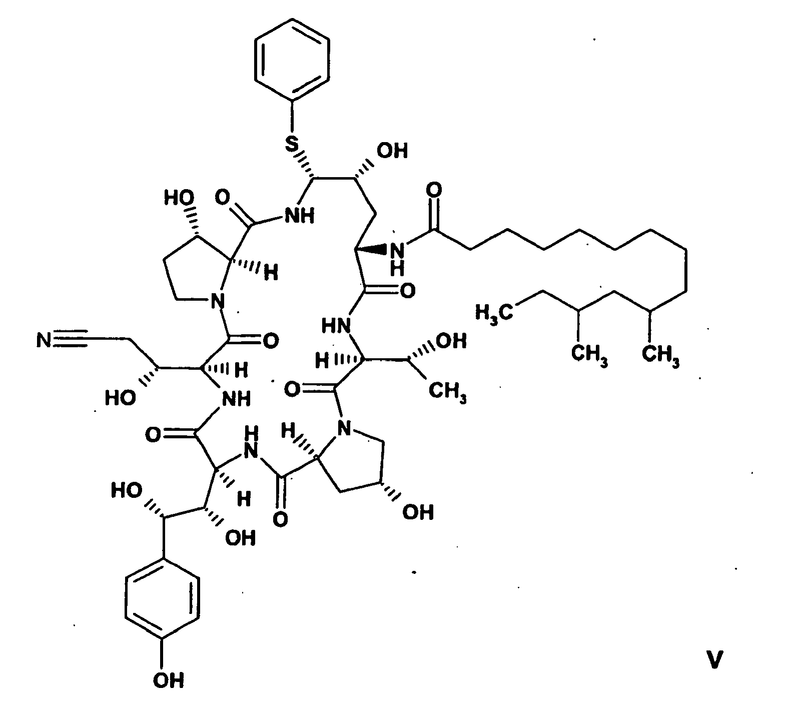
c) reacting the compound of formula V as obtained in step b) with H₂N-CH₂-CH₂-NH₂ to obtain a compound of formula VI or an acid addition salt or a solvate thereof, and
d) optionally isolating the compound of formula VI or an acid addition salt or a solvate thereof as obtained in step c).

12. A process according to claim 11 wherein the compound of formula VI in step d) is isolated in its monoacetate salt form.

13. A process according to any one of claims 5 to 12 wherein cyanuric chloride is used as dehydrating agent in step a).

14. Use of a compound according to any of claims 1 to 3 in the preparation of caspofungin.

## Patentansprüche

1. Verbindung der Formel II 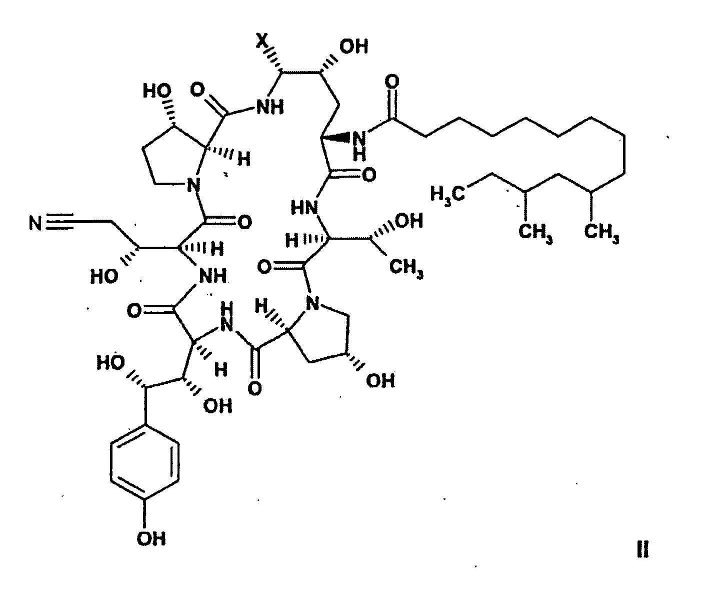 oder ein Säureadditionssalz oder ein Solvat davon, wobei X NR₁R₂ ist, und wobei
R₁ H, C₁-C₈-Alkyl, C₃-C₄-Alkenyl, (CH₂)₂₋₄OH oder (CH₂)₂₋₄NR₃R₄ ist;
R₂ H, C₁-C₈-Alkyl, C₃-C₄-Alkenyl, (CH₂)₂₋₄OH, (CH₂)₂₋₄NR₃R₄ ist; oder
wobei NR₁R₂ einen heterozyklischen Ring bildet und R₁ und R₂ zusammen (CH₂)₄, (CH₂)₅, (CH₂)₂O(CH₂)₂ oder (CH₂)₂NH(CH₂)₂ sind;
R₃ H oder C₁-C₈-Alkyl ist;
R₄ H oder C₁-C₈-Alkyl ist.

2. Verbindung nach Anspruch 1 der Formel VI 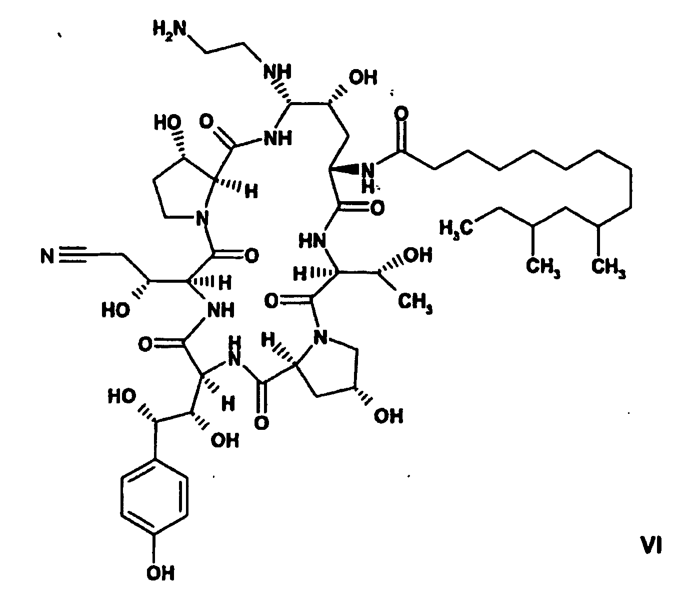 oder ein Säureadditionssalz oder ein Solvat davon.

3. Verbindung nach Anspruch 1 oder 2 der Formel VIa

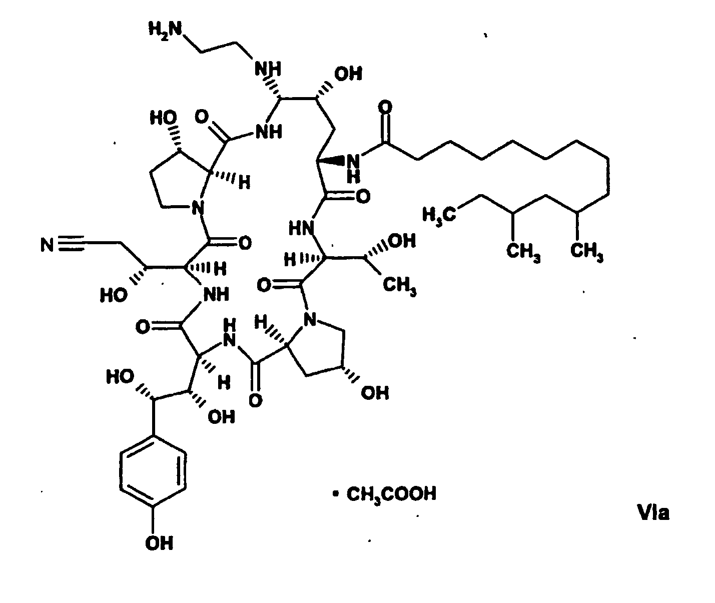

4. Verfahren zur Herstellung von Aza-cyclohexapeptid-Verbindungen der Formel I 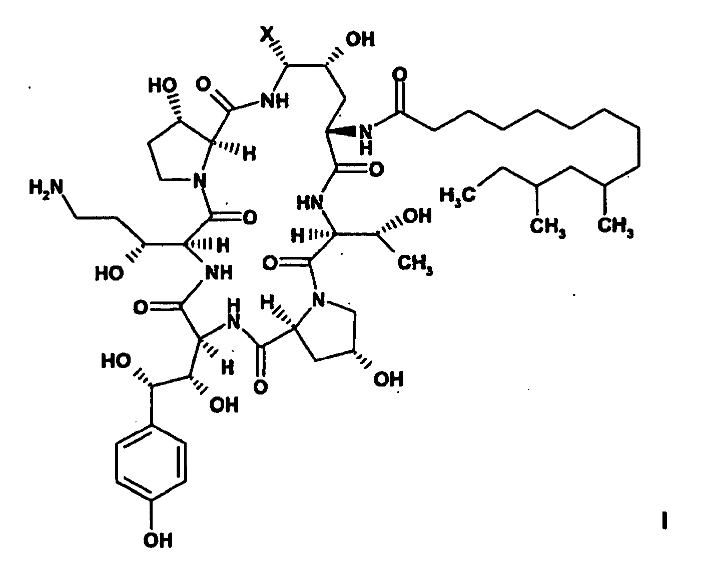 wobei X NR₁R₂ ist und wobei
R₁ H, C₁-C₈-Alkyl, C₃-C₄-Alkenyl, (CH₂)₂₋₄OH oder (CH₂)₂₋₄NR₃R₄ ist;
R₂ H, C₁-C₈-Alkyl, C₃-C₄-Alkenyl, (CH₂)₂₋₄OH, (CH₂)₂₋₄NR₃R₄ ist; oder
wobei NR₁R₂ einen heterozyklischen Ring bildet und R₁ und R₂ zusammen (CH₂)₄, (CH₂)₅, (CH₂)₂O(CH₂)₂ oder (CH₂)₂NH(CH₂)₂ sind;
R₃ H oder C₁-C₈-Alkyl ist;
R₄ H oder C₁-C₈-Alkyl ist.
oder eines pharmazeutisch verträglichen Salzes davon, wobei das Verfahren die Schritte umfasst:
a) Reduzieren einer Verbindung der Formel II oder eines Säureadditionssalzes davon 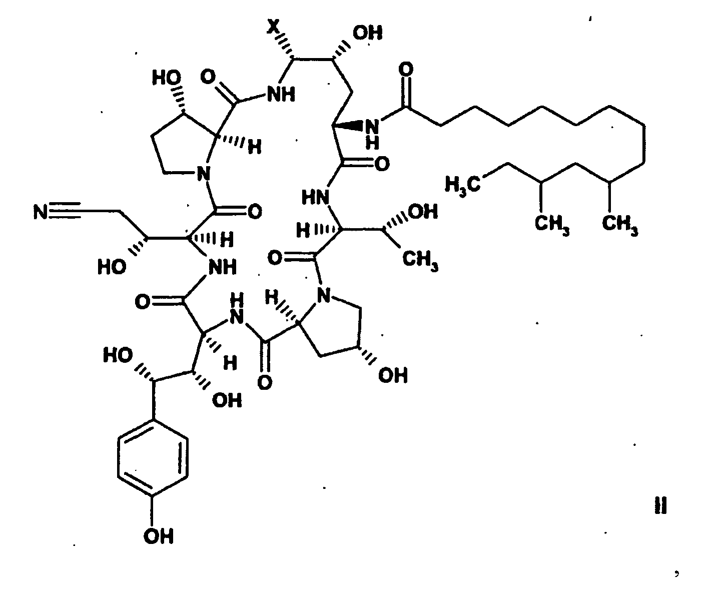 wobei X wie vorstehend definiert ist, um eine Verbindung der Formel I oder eines pharmazeutisch verträglichen Salzes davon zu erhalten, und
b) gegebenenfalls Isolieren der wie in Schritt a) erhaltenen Verbindung der Formel I oder eines pharmazeutisch verträglichen Salzes davon.

5. Verfahren nach Anspruch 4 zur Herstellung der wie in Anspruch 4 definierten Aza-cyclohexapeptid-Verbindungen der Formel I
oder eines pharmazeutisch verträglichen Salzes davon, wobei das Verfahren die Schritte umfasst:
a) Umsetzen einer Verbindung der Formel III 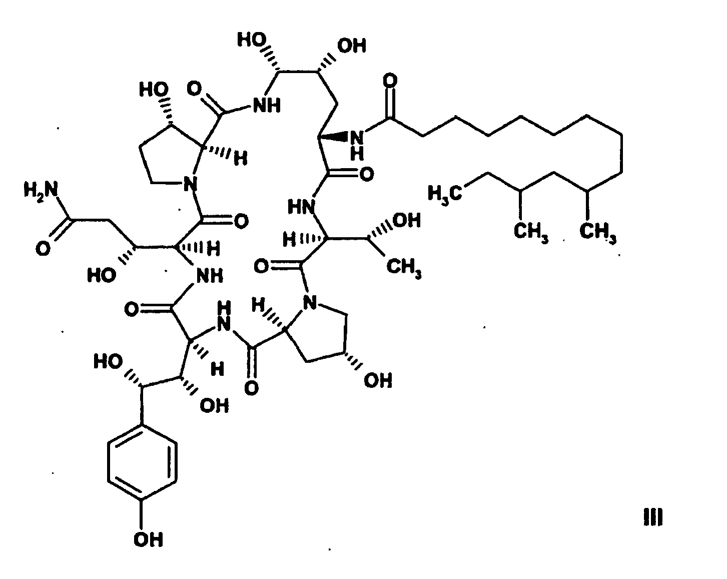 mit einem Dehydratisierungsmittel, um eine Verbindung der Formel IV zu erhalten 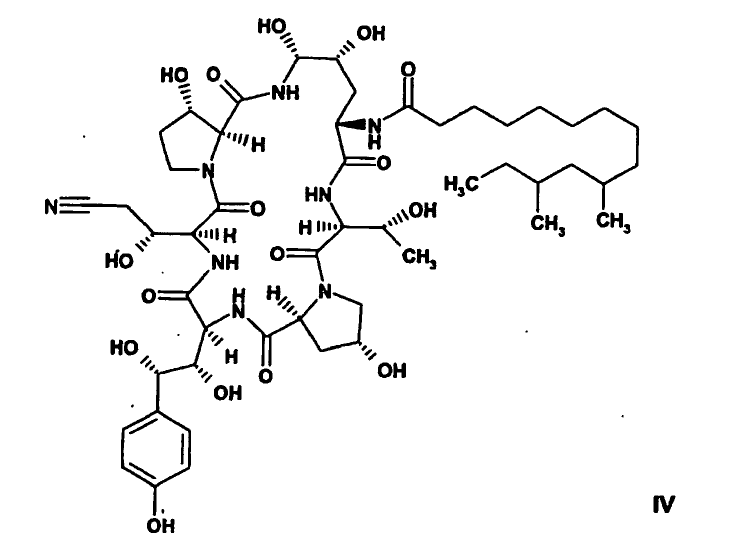
b) Umsetzen der wie in Schritt a) erhaltenen Verbindung der Formel IV mit einem Thiophenol, um eine Verbindung der Formel V zu erhalten 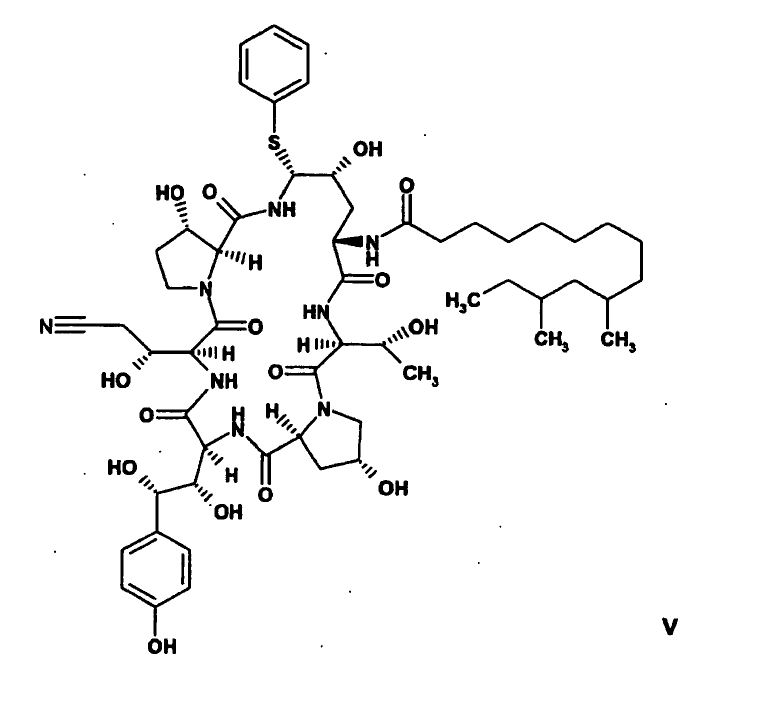
c) Umsetzen der wie in Schritt b) erhaltenen Verbindung der Formel V mit einer Verbindung der Formel HX, wobei X wie in Anspruch 4 definiert ist, um eine wie in Anspruch 1 definierte Verbindung der Formel II oder ein Säureadditionssalz davon zu erhalten,
d) Reduzieren einer wie in Schritt c) erhaltenen Verbindung der Formel II oder eines Säureadditionssalzes davon, um eine Verbindung der Formel I oder ein pharmazeutisch verträgliches Salzes davon zu erhalten, und
e) gegebenenfalls Isolieren der wie in Schritt d) erhaltenen Verbindung der Formel I oder eines pharmazeutisch verträglichen Salzes davon.

6. Verfahren nach Anspruch 4 oder 5, wobei X HN-CH₂-CH₂-NH₂ ist.

7. Verfahren nach Anspruch 4 oder 5, wobei der Reduktionsschritt durch katalytische Hydrierung durchgeführt wird.

8. Verfahren nach Anspruch 7, wobei Rh/Al₂O₃ oder Pd auf Kohlenstoff als Katalysator verwendet wird.

9. Verfahren nach einem beliebigen der Ansprüche 4 bis 8, wobei in dem Reduktionsschritt H₂ als Wasserstoffquelle verwendet wird.

10. Verfahren zur Herstellung einer Verbindung der Formel II gemäß Anspruch 1 oder eines Säureadditionssalz oder eines Solvat davon, wobei das Verfahren die Schritte umfasst:
a) Umsetzen einer Verbindung der Formel III 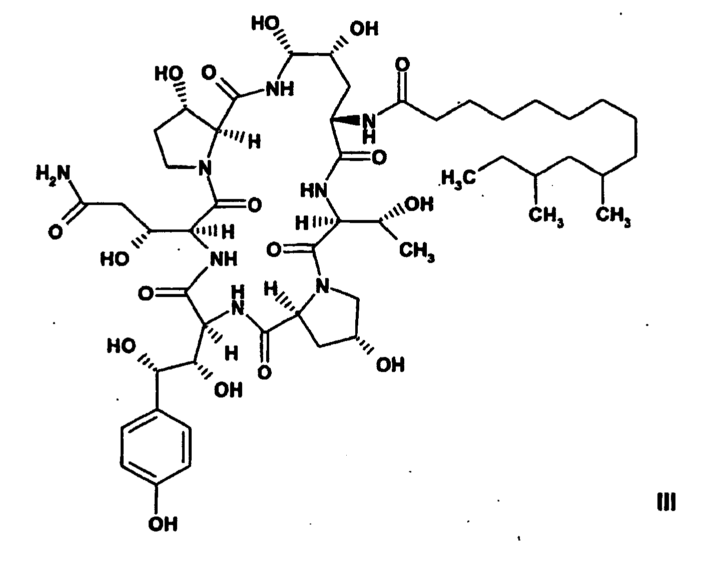 mit einem Dehydratisierungsmittel, um eine Verbindung der Formel IV zu erhalten 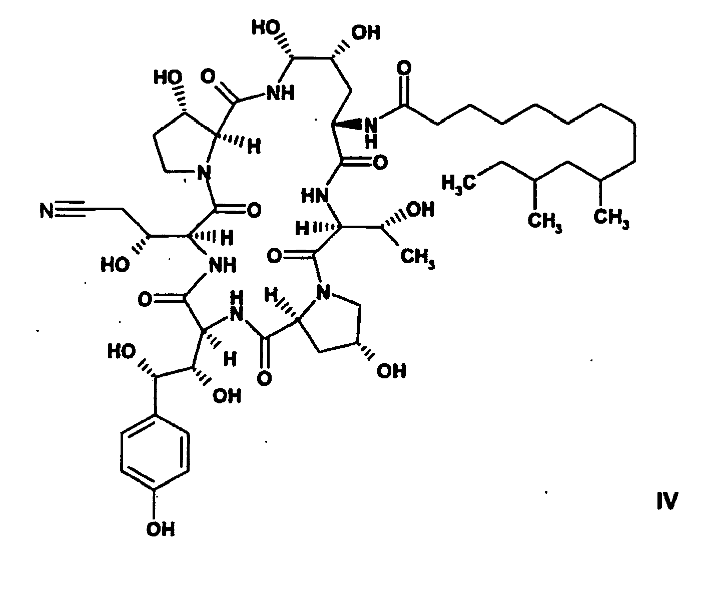
b) Umsetzen der wie in Schritt a) erhaltenen Verbindung der Formel IV mit einem Thiophenol, um eine Verbindung der Formel V zu erhalten 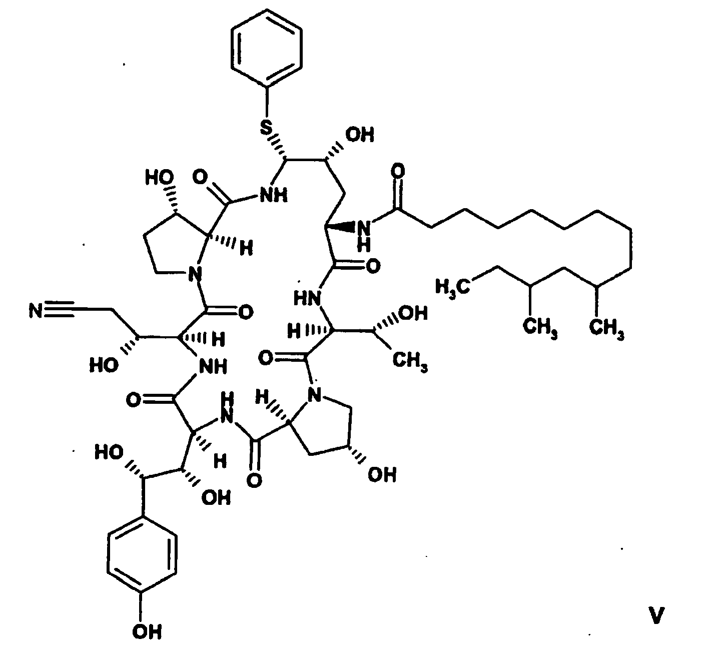
c) Umsetzen der wie in Schritt b) erhaltenen Verbindung der Formel V mit einer Verbindung der Formel HX, wobei X NR₁R₂ ist, und wobei
R₁ H, C₁-C₈-Alkyl, C₃-C₄-Alkenyl, (CH₂)₂₋₄OH oder (CH₂)₂₋₄NR₃R₄ ist;
R₂ H, C₁-C₈-Alkyl, C₃-C₄-Alkenyl, (CH₂)₂₋₄OH, (CH₂)₂₋₄NR₃R₄ ist; oder
wobei NR₁R₂ einen heterozyklischen Ring bildet und R₁ und R₂ zusammen (CH₂)₄, (CH₂)₅, (CH₂)₂O(CH₂)₂ oder (CH₂)₂NH(CH₂)₂ sind;
R₃ H oder C₁-C₈-Alkyl ist;
R₄ H oder C₁-C₈-Alkyl ist;
um eine Verbindung der Formel II oder ein Säureadditionssalz oder ein Solvat davon zu erhalten, und
d) gegebenenfalls Isolieren der wie in Schritt c) erhaltenen Verbindung der Formel II oder eines Säureadditionssalzes oder eines Solvates davon.

11. Verfahren nach Anspruch 10 zur Herstellung einer Verbindung der Formel VI gemäß Anspruch 2 oder eines Säureadditionssalzes oder eines Solvates davon, wobei das Verfahren die Schritte umfasst:
a) Umsetzen einer Verbindung der Formel III 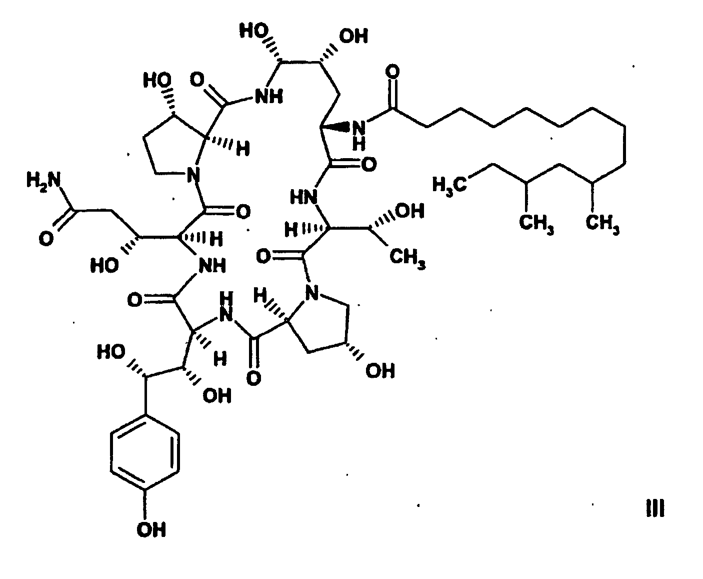 mit einem Dehydratisierungsmittel, um eine Verbindung der Formel IV zu erhalten 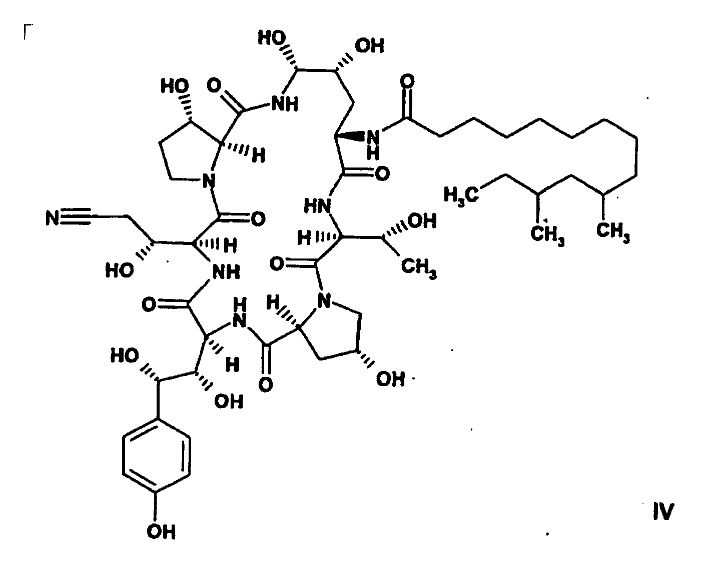
b) Umsetzen der wie in Schritt a) erhaltenen Verbindung der Formel IV mit einem Thiophenol, um eine Verbindung der Formel V zu erhalten 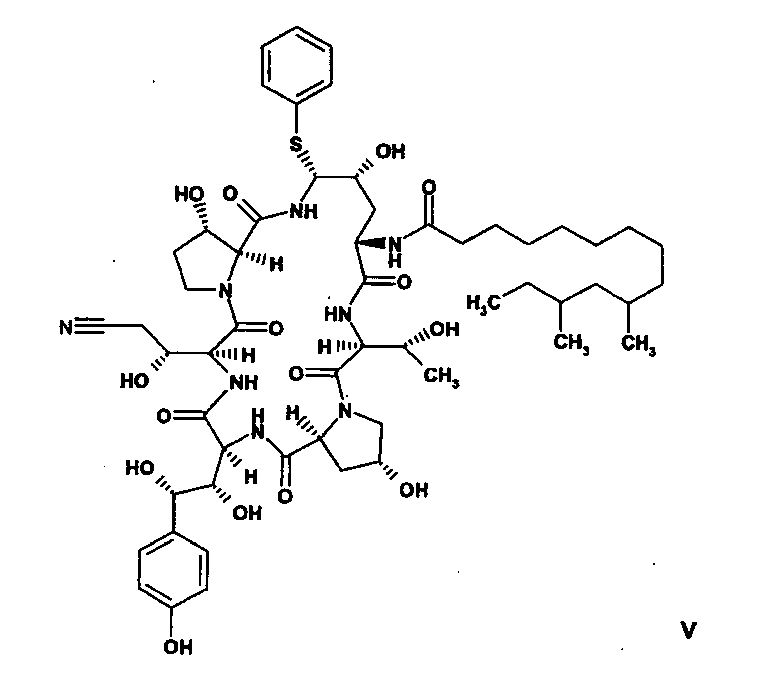
c) Umsetzen der wie in Schritt b) erhaltenen Verbindung der Formel V mit H₂N-CH₂-CH₂-NH₂, um eine Verbindung der Formel VI oder ein Säureadditionssalz oder ein Solvat davon zu erhalten, und
d) gegebenenfalls Isolieren der wie in Schritt c) erhaltenen Verbindung der Formel VI oder eines Säureadditionssalzes oder eines Solvates davon.

12. Verfahren nach Anspruch 11, wobei die Verbindung der Formel VI in Schritt d) in Form seines Monoacetat-Salzes isoliert wird.

13. Verfahren nach einem beliebigen der Ansprüche 5 bis 12, wobei Cyanurchlorid als Dehydratisierungsmittel in Schritt a) verwendet wird.

14. Verwendung einer Verbindung nach einem beliebigen der Ansprüche 1 bis 3 zur Herstellung von Caspofungin.

## Revendications

1. Composé de formule II 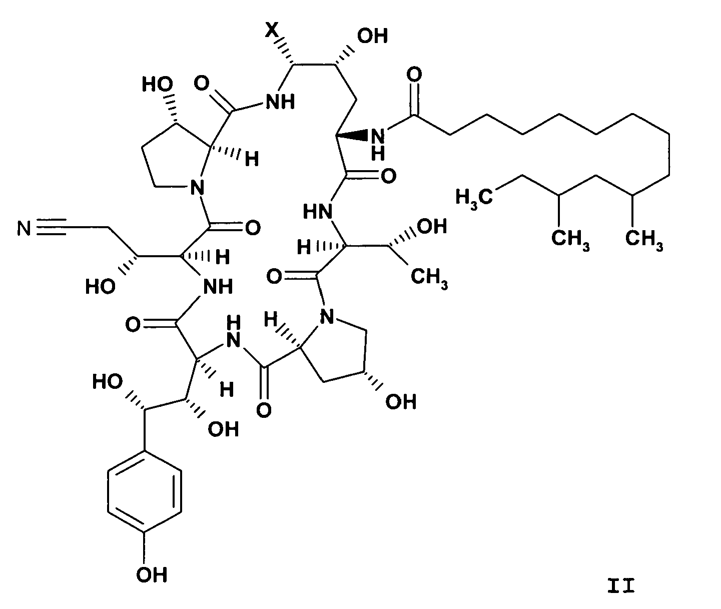 ou sel d'addition d'acide ou solvate de celui-ci, dans lequel X est NR₁R₂, et dans lequel
R₁ est H, un alkyle en C₁-C₈, un alcényle en C₃-C₄, (CH₂)₂₋₄OH ou (CH₂)₂₋₄NR₃R₄;
R₂ est H, un alkyle en C₁-C₈, un alcényle en C₃-C₄, (CH₂)₂₋₄OH, (CH₂)₂₋₄NR₃R₄; ou
dans lequel NR₁R₂ forme un cycle hétérocyclique et R₁ et R₂ ensemble sont (CH₂)₄, (CH₂)₅, (CH₂)₂O(CH₂)₂ ou (CH₂)₂NH(CH₂)₂ ;
R₃ est H ou un alkyle en C₁-C₈ ;
R₄ est H ou un alkyle en C₁-C₈.

2. Composé selon la revendication 1, de formule VI 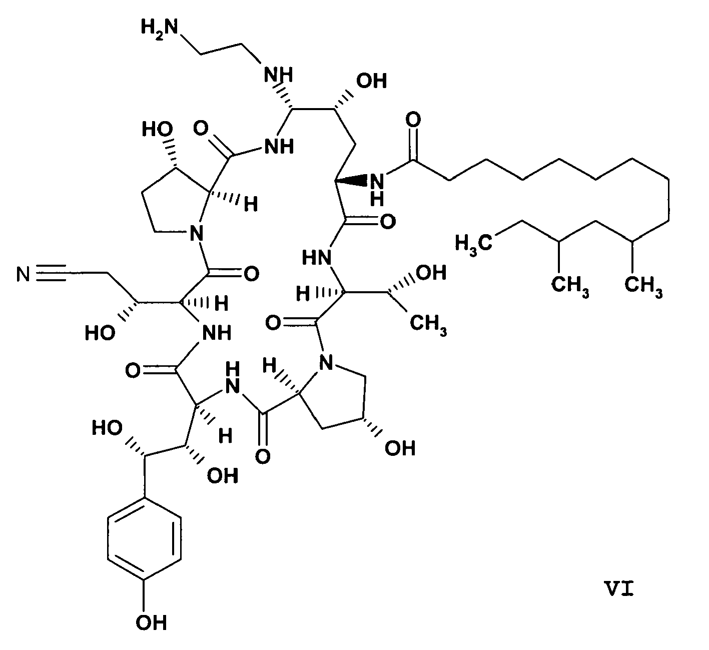 ou sel d'addition d'acide ou solvate de celui-ci.

3. Composé selon la revendication 1 ou 2, de formule VIa

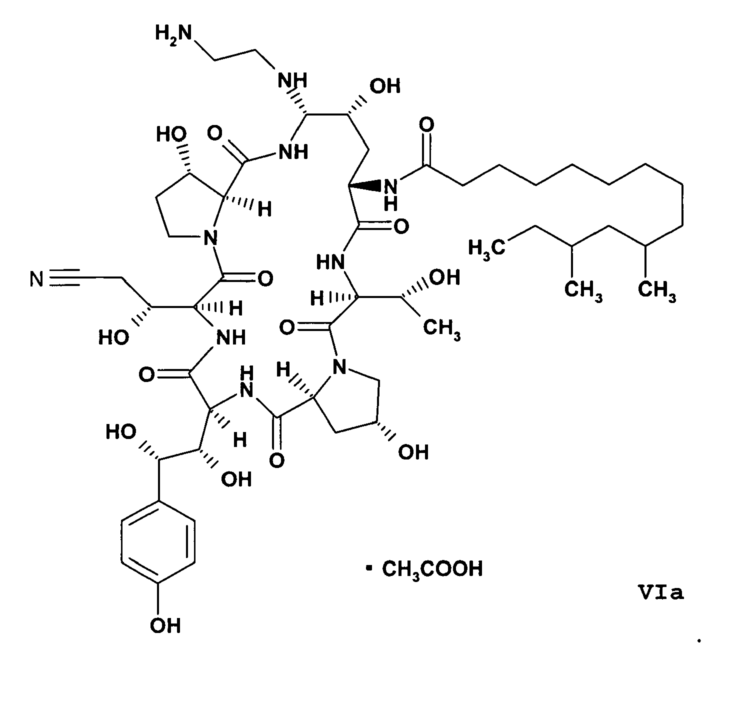

4. Procédé de production de composés azacyclohexapeptidiques de formule 1 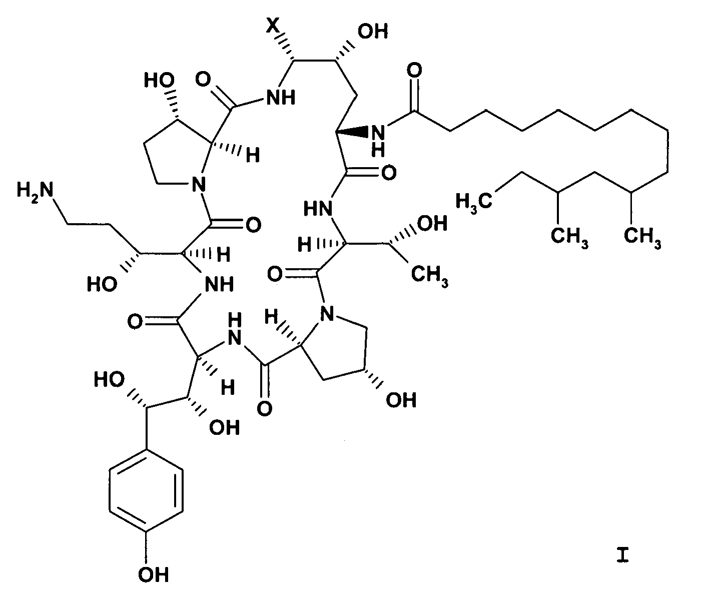 dans lesquels X est NR₁R₂ et dans lesquels
R₁ est H, un alkyle en C₁-C₈, un alcényle en C₃-C₄, (CH₂)₂₋₄OH ou (CH₂)₂₋₄NR₃R₄ ;
R₂ est H, un alkyle en C₁-C₈, un alcényle en C₃-C₄, (CH₂)₂₋₄OH, (CH₂)₂₋₄NR₃R₄ ; ou
dans lesquels NR₁R₂ forme un cycle hétérocyclique et R₁ et R₂ ensemble sont (CH₂)₄, (CH₂)₅, (CH₂)₂O(CH₂)₂ ou (CH₂)₂NH(CH₂)₂ ;
R₃ est H ou un alkyle en C₁-C₈ ;
R₄ est H ou un alkyle en C₁-C₈ ;
ou d'un sel pharmaceutiquement acceptable de ceux-ci, comprenant les étapes qui consistent à :
a) réduire un composé de formule II ou un sel d'addition d'acide de celui-ci 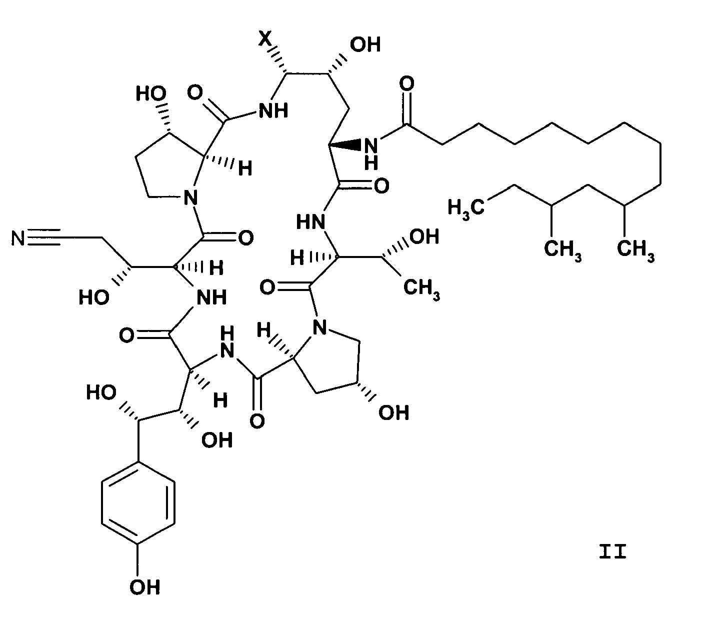 dans lequel X est tel que défini ci-dessus, afin d'obtenir un composé de formule I ou un sel pharmaceutiquement acceptable de celui-ci, et
b) éventuellement, isoler le composé de formule I ou un sel pharmaceutiquement acceptable de celui-ci obtenu à l'étape a).

5. Procédé selon la revendication 4 pour la production de composés azacyclohexapeptidiques de formule I tels que définis dans la revendication 4 ou d'un sel pharmaceutiquement acceptable de ceux-ci, comprenant les étapes qui consistent à :
a) faire réagir un composé de formule III 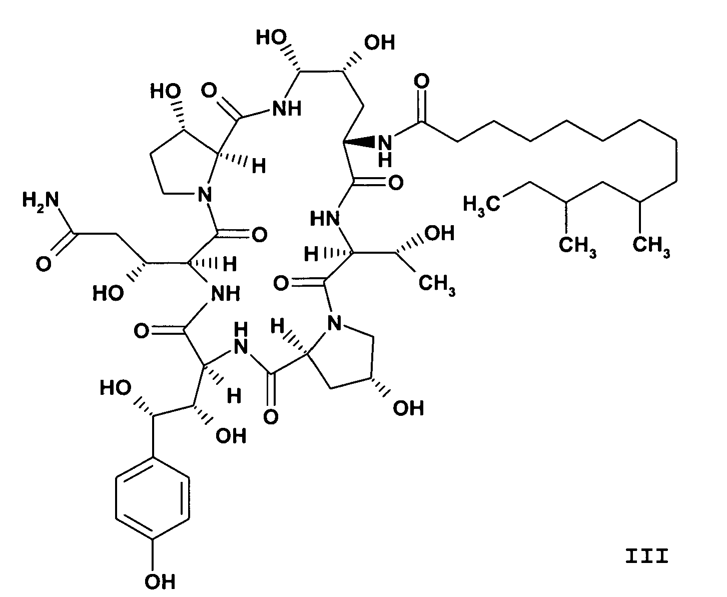 avec un agent déshydratant afin d'obtenir un composé de formule IV 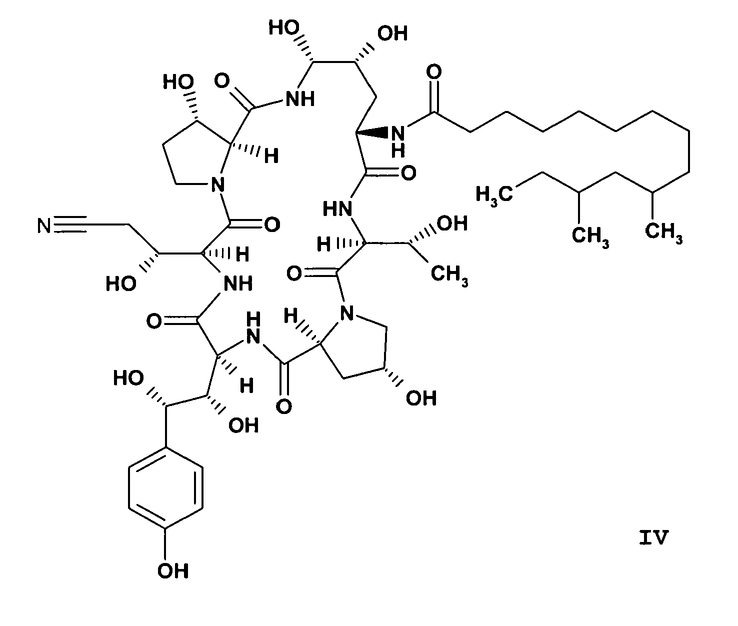
b) faire réagir le composé de formule IV obtenu à l'étape a) avec un thiophénol afin d'obtenir un composé de formule V 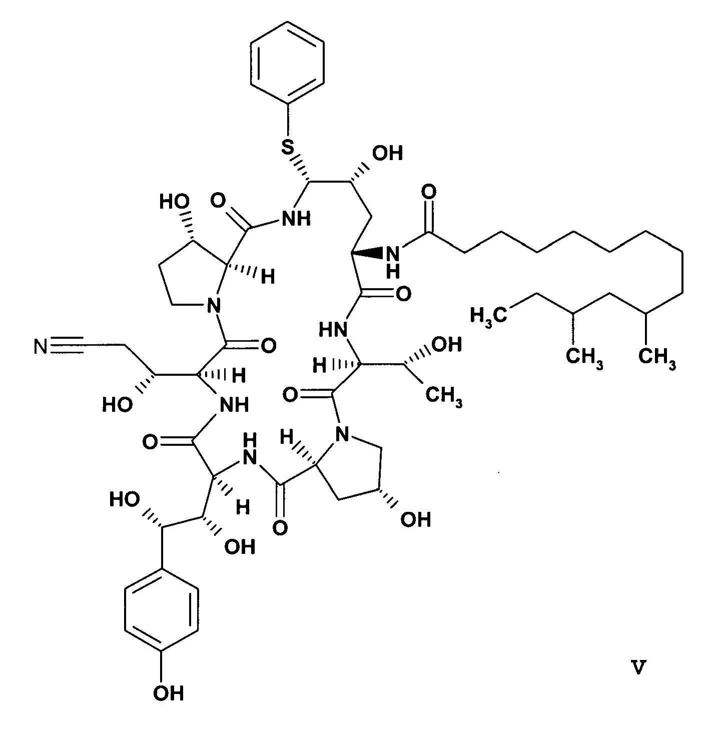
c) faire réagir le composé de formule V obtenu à l'étape b) avec un composé de formule HX, dans lequel X est tel que défini dans la revendication 4, afin d'obtenir un composé de formule II tel que défini dans la revendication 1 ou un sel d'addition d'acide de celui-ci,
d) réduire un composé de formule II ou un sel d'addition d'acide de celui-ci obtenu à l'étape c) afin d'obtenir un composé de formule I ou un sel pharmaceutiquement acceptable de celui-ci, et
e) éventuellement, isoler le composé de formule I ou un sel pharmaceutiquement acceptable de celui-ci obtenu à l'étape d).

6. Procédé selon la revendication 4 ou 5, dans lequel X est HN-CH₂-CH₂-NH₂.

7. Procédé selon la revendication 4 ou 5, dans lequel l'étape de réduction est réalisée par hydrogénation catalytique.

8. Procédé selon la revendication 7, dans lequel on utilise, comme catalyseur, du Rh/Al₂O₃ ou du Pd sur charbon.

9. Procédé selon l'une quelconque des revendications 4 à 8, dans lequel, lors de l'étape de réduction, on utilise de l'H₂ comme source d'hydrogène.

10. Procédé de production d'un composé de formule II selon la revendication 1 or d'un sel d'addition d'acide ou d'un solvate de celui-ci, comprenant les étapes qui consistent à :
a) faire réagir un composé de formule III 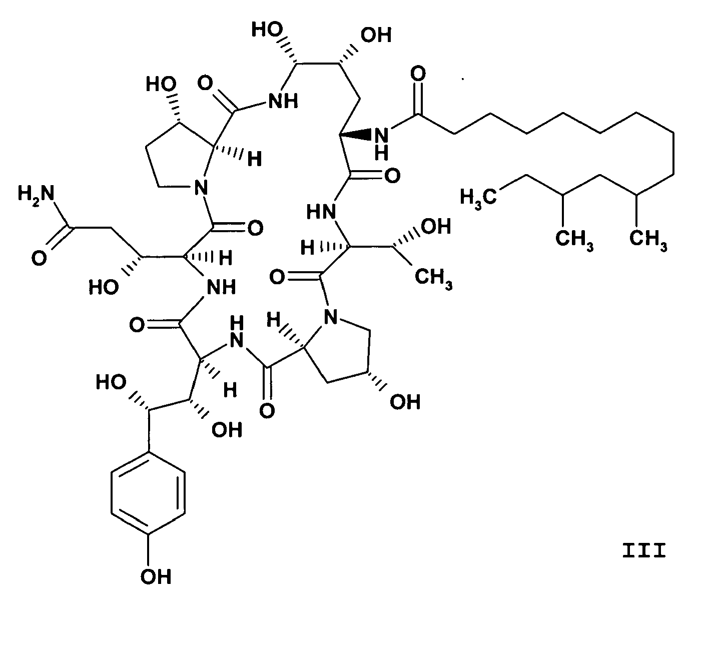 avec un agent déshydratant afin d'obtenir un composé de formule IV 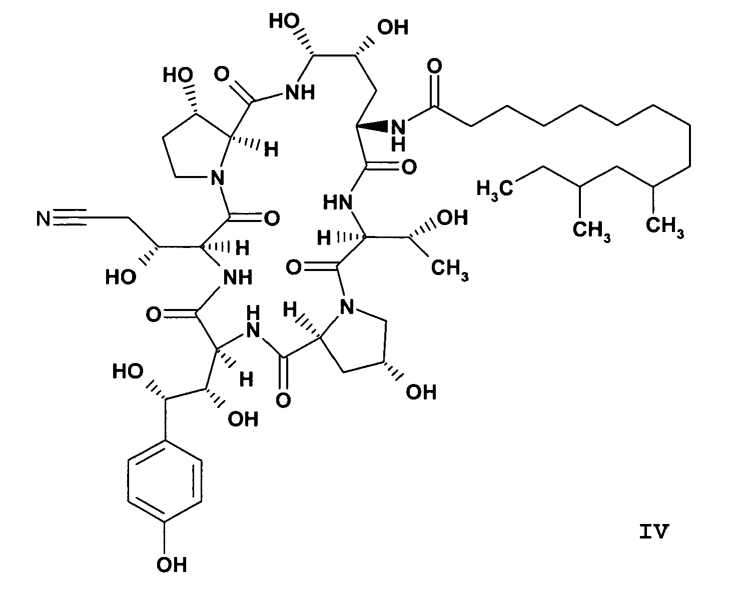
b) faire réagir le composé de formule IV obtenu à l'étape a) avec un thiophénol afin d'obtenir un composé de formule V 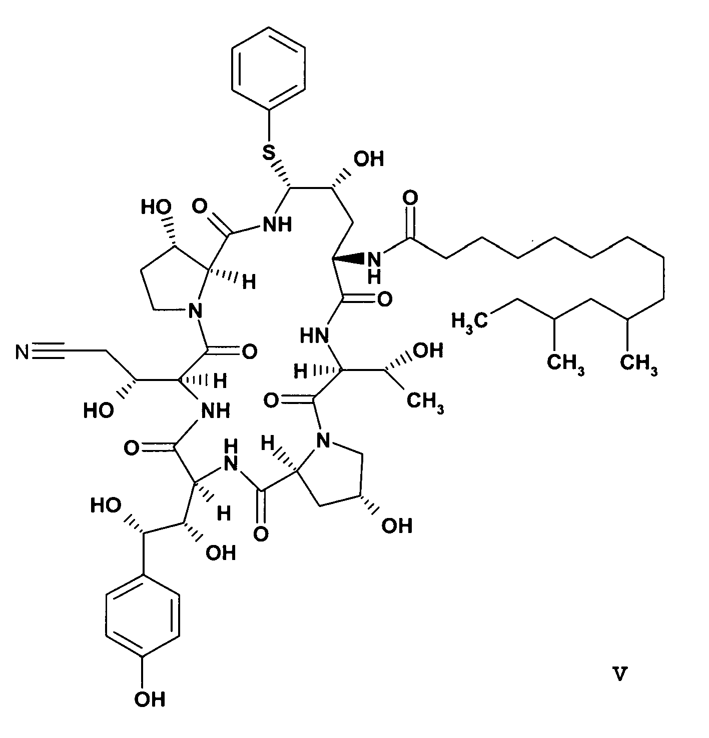
c) faire réagir le composé de formule V obtenu à l'étape b) avec un composé de formule HX, dans lequel X est NR₁R₂, et dans lequel
R₁ est H, un alkyle en C₁-C₈, un alcényle en C₃-C₄, (CH₂)₂₋₄OH ou (CH₂)₂₋₄NR₃R₄;
R₂ est H, un alkyle en C₁-C₈, un alcényle en C₃-C₄, (CH₂)₂₋₄OH, (CH₂)₂₋₄NR₃R₄ ; ou
dans lequel NR₁R₂ forme un cycle hétérocyclique et R₁ et R₂ ensemble sont (CH₂)₄, (CH₂)₅, (CH₂)₂O(CH₂)₂ ou (CH₂)₂NH(CH₂)₂ ;
R₃ est H ou un alkyle en C₁-C₈ ;
R₄ est H ou un alkyle en C₁-C₈ ;
afin d'obtenir un composé de formule II ou un sel d'addition d'acide ou un solvate de celui-ci, et
d) éventuellement, isoler le composé de formule II ou un sel d'addition d'acide ou un solvate de celui-ci obtenu à l'étape c).

11. Procédé selon la revendication 10 pour la production d'un composé de formule VI selon la revendication 2 ou d'un sel d'addition d'acide ou d'un solvate de celui-ci, comprenant les étapes qui consistent à :
a) faire réagir un composé de formule III 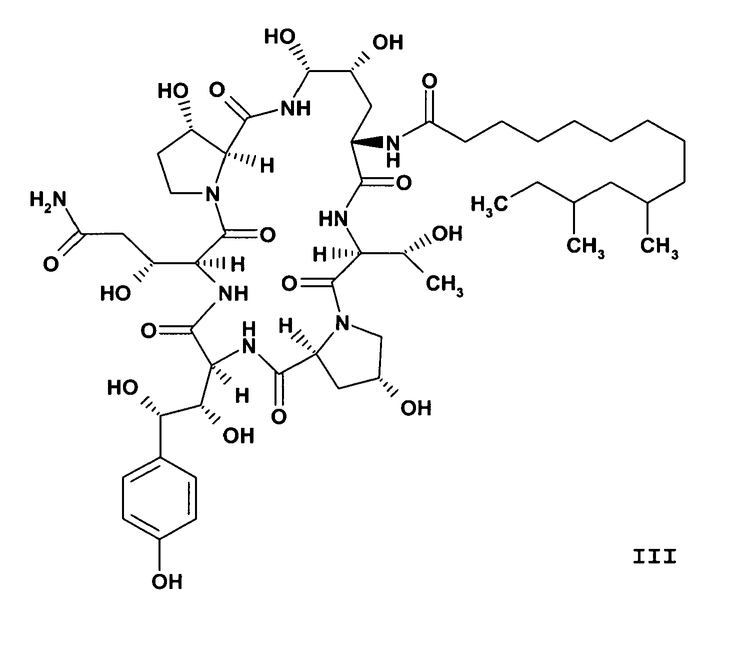 avec un agent déshydratant afin d'obtenir un composé of formule IV 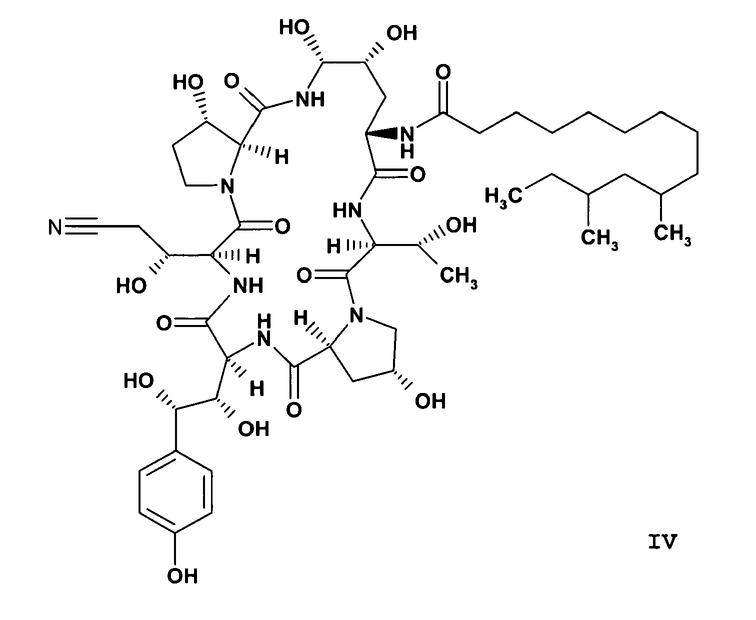
b) faire réagir le composé de formule IV obtenu à l'étape a) avec un thiophénol afin d'obtenir un composé de formule V, 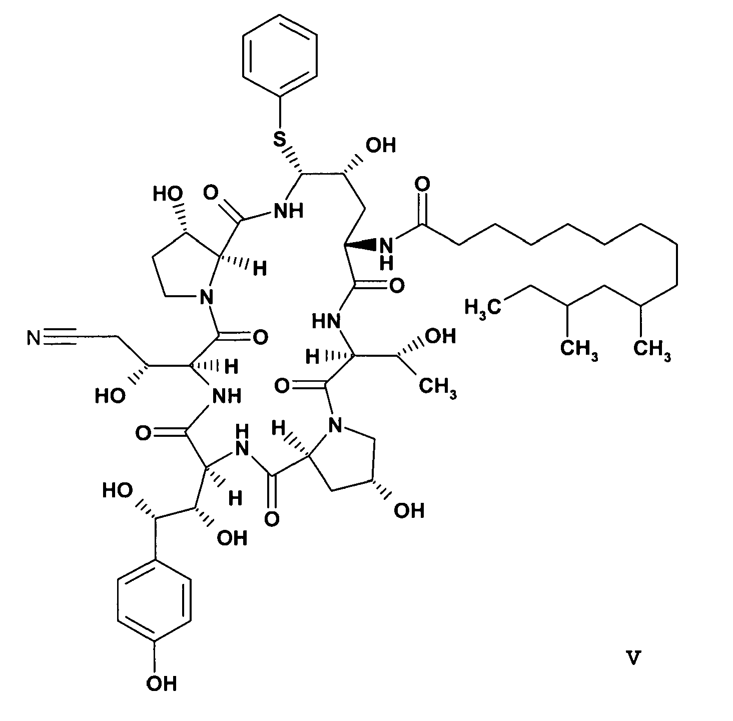
c) faire réagir le composé de formule V obtenu à l'étape b) avec du H₂N-CH₂-CH₂-NH₂ afin d'obtenir un composé de formule VI ou un sel d'addition d'acide ou un solvate de celui-ci, et
d) éventuellement, isoler le composé de formule VI ou un sel d'addition d'acide ou un solvate de celui-ci obtenu à l'étape c).

12. Procédé selon la revendication 11, dans lequel le composé de formule VI à l'étape d) est isolé en sa forme de sel monoacétate.

13. Procédé selon l'une quelconque des revendications 5 à 12, dans lequel on utilise du chlorure cyanurique en tant qu'agent déshydratant à l'étape a).

14. Utilisation d'un composé selon l'une quelconque des revendications 1 à 3 dans la préparation de caspofungine.
